# EUROPÄISCHE PATENTANMELDUNG

(11) **EP 2 778 154 A1**
(43) Veröffentlichungstag der Anmeldung: **17.09.2014**
(21) Anmeldenummer: 13158985.5
(22) Anmeldetag: 13.03.2013
(51) Int. Cl.: C07D 207/48, C07C 67/475, C07C 41/18, C07C 6/04

(54) **In situ Generierung von Ruthenium-Katalysatoren zur Olefin-Metathese**

(71) Anmelder: Evonik Industries AG, 45128 Essen (DE)
(72) Erfinder: Kadyrov,Renat, 60389 Frankfurt (DE); Luebbe,Christa, 67346 Speyer (DE); Dumrath,Andreas, 67346 Speyer (DE); Neumann,Helfried, 18055 Rostock (DE); Beller,Matthias, 18211 Niehagen (DE)

(57) **Zusammenfassung**

Die vorliegende Erfindung betrifft ein Verfahren zur Herstellung von Olefinen mittels Metathese umfassend die folgenden Schritte
a. Bereitstellung einer Olefin-Reaktionsmischung enthaltend mindestens ein Olefin,
b. Zugabe einer Ruthenium-Verbindung der allgemeinen Formel [RuX₂L¹ₓL²_{y}]_{z} (I), wobei X = anionischer Ligand; L¹ = neutraler π-bindender Ligand; L² = neutraler Elektronendonor-Ligand; x = 0, 1; y = 1, 2,3; z = 1, 2,
c. Zugabe einer Lewissäure und/oder eines anionischen, nicht-koordinierenden Salzes,
d. Umsetzung bei Temperaturen in einem Bereich von 30°C bis 140°C,
wobei keine Zugabe eines Alkins oder Alkinols erfolgt.

Die Erfindung betrifft weiterhin die Verwendung dieses Verfahrens in Metathesereaktionen.

## Beschreibung

Die vorliegende Erfindung betrifft ein Verfahren zur Herstellung von Olefinen mittels Metathese in Gegenwart von "in situ" generierten Ru-Katalysatorkomplexen.

In den letzten Jahrzehnten hat sich die Olefin-Metathese als eine wirksame Kohlenstoff-Kohlenstoff-Verknüpfungsreaktion durchgesetzt und findet breite Anwendung in der organischen Synthese und Polymerwissenschaft. Dabei haben vor allem Ruthenium-Carben-Katalysatoren und ihre Derivate die Olefin-Metathese als eine vielseitige und zuverlässige Synthesemethode fest in der anspruchsvollen organischen Synthese verankert. Die ausgesprochen große Substratbreite und außerdem große Toleranz gegenüber verschiedensten funktionellen Gruppen macht die Olefin-Metathese zu einer nützlichen, schnellen und effizienten Synthesetechnik für sonst über traditionelle organische Synthese nur schwer zugängliche Moleküle. Eine Vielzahl von verschiedenen Übergangsmetallkomplexen kann als Katalysatoren in der Olefin-Metathese dienen. Insbesondere bestimmte Ruthenium- und Osmium-Carben-Verbindungen zeichnen sich als effektive Katalysatoren in Olefin-Metathese-Reaktionen wie zum Beispiel der Kreuzmetathese (CM), der Ringschlussmetathese (RCM), der Ringöffnenden Metathese (ROM), der Ringöffnenden Metathesepolymerisation (ROMP), oder der Acyclischen Dien-Metathese (ADMET) aus.

Besonders aktive Olefin-Metathese-Katalysatoren tragen zumeist sowohl Phosphan-Liganden als auch nucleophile heterocyclische Carben-Liganden (NHC-Liganden) und besitzen zusätzlich eine Metall-Carben-Struktur. Ein prominentes Beispiel für diese Art der Olefin-Metathese-Katalysatoren sind die zum Teil kommerziell erhältlichen Ru-Komplexe der allgemeinen Struktur RuX₂(=CR₂)LL', wobei X ein anionischer Ligand ist, R ausgewählt wird aus der Gruppe bestehend aus Wasserstoff, (C₆-C₁₄)-Aryl- und (C₃-C₁₄) -Heteroarylrest, und L und L' einen neutralen Elektronendonor-Liganden bezeichnen, wobei L ein N-heterocyclisches Carben und L' ein Phosphan ist.

Die Synthese dieser Ruthenium-Carben-Komplexe ist vergleichsweise aufwendig, rohstoffintensiv und teuer. Typische Syntheseprotokolle bestehen aus mehreren Stufen, wobei zum Teil aufwendige Prozessführung unter Inertgasatmosphäre, schwer zugängliche Ausgangsstoffe oder die Nutzung sicherheitsrelevanter Reaktionspartner benötigt werden. Insbesondere die Nutzung von Carben-Vorstufen, wie disubstituierte Cyclopropene (WO 93/20111), Diazoalkane (WO 97/06185) oder Acetylene (DE 19854869) stellen in technischem Maßstab ein erhebliches Sicherheitsrisiko dar und sollten demzufolge vermieden werden. Metallorganische Startmaterialen wie RuCl₂(PPh₃)₃ (Hill et al., Dalton 1999, 285-291) oder RuHCl(PPh₃)₃ (Hoffmann et al., Journal of Organometallic Chemistry 2002, 641, 220-226) werden unter Einsatz eines großen Überschusses an Triphenylphosphan (PPh₃) aus RuCl₃ hergestellt, wobei diese PPh₃-Liganden bei der anschließenden Katalysatorsynthese durch eine Ligandenaustauschreaktion verloren gehen. Neueste Syntheseverbesserungen können diesen Nachteil zum Teil durch die direkte Umsetzung von Tricyclohexylphosphan mit Rutheniumchlorid-Hydrat oder Ru(cod)Cl₂ zu den (PCy₃)₂RuCl₂-Carben-Komplexen kompensieren (WO 2009/124977). Es werden jedoch weiterhin große Mengen an PCy₃ oder PCy₃-Lösung benötigt. Außerdem wird bei der Synthese der 2. Generation von Ruthenium-Carben-Komplexen einer dieser PCy₃-Liganden durch einen neutralen Elektronendonor-Liganden ausgetauscht, wodurch wiederum PCy₃ aus dem Komplex verloren geht. Preiswertere Alternativen zu Ruthenium-Phosphan-Komplexen sind neutrale oder anionische Ruthenium-Aryl-Komplexe nach der allgemeinen Struktur [RuX(=C=[C]ₙ=CR₂)L¹L²]Y (EP0921129A1). X und R sind wie oben beschrieben definiert, Y sind anionische, schwach koordinierende Liganden, L¹ sind Phosphane, Phosphite, Phosphonite, Phosphinite, Arsane oder Stibene, L² ist Benzol oder ein substituiertes Benzolderivat, wie zum Beispiel p-Cymene. Nachteilig ist, dass auch für die Aktivität der Ruthenium-Aryl-Komplexe die Struktureinheit Ru=C=[C]ₙ=CR₂ relevant ist, welche wiederum die Nutzung von gefährlichen, schwer zugänglichen oder sehr empfindlichen Carben-Vorstufen voraussetzt.

Es ist bereits eine Vielzahl von Beispielen für "in situ" generierte katalytische Systeme aus verfügbaren Ruthenium-Vorstufen als alternativer Ansatz beschrieben worden. Zur Erzeugung des aktiven Katalysators wird dabei jedoch entweder
1) eine photochemische Aktivierung (A. Hafner, A. Mühlebach, P. A. van der Schaaf, Angew. Chem. 1997, 109, 2213-2216; Angew. Chem. Int. Ed., 1997, 36, 2213-2216., L. Delaude, A. Demonceau and A. F. Noels, Chem. Commun., 2001, 986-987., A. Fürstner, and L. Ackermann, Chem. Commun., 1999, 95-96, L. Jafarpour, J. Huang, E. D. Stevens, and S. P. Nolan Organometallics 1999, 18, 3760-3763)
2) eine Aktivierung über Carben-Vorstufen wie Trimethylsilyldiazomethan (a) A. W. Stumpf, E. Saive, A. Demonceau and A. F. Noels, Chem. Commun., 1995, 1127; b) A. Demonceau, A. W. Stumpf, E. Saive and A. F. Noels, Macromolecules, 1997, 30, 2127), oder
3) die Aktivierung mit Alkinen (Y. Miyaki, T. Onishi, S. Ogoshi, H. Kurosawa, J. Organometallic Chem. 2000, 616, 135-139., J. Louie, R. H. Grubbs, Angew. Chem. Int. Ed. 2001, 40, 247-9., D. Sémeril, C. Bruneau, P. H. Dixneuf, Helv. Chim. Cata 2001, 84, 3335- 3341; D. Sémeril, C. Bruneau, P. H. Dixneuf Adv. Synth. Catal. 2002, 344, 585-595) verwendet.

Es wurde auch berichtet, dass homobimetallische Ru-NHC-Komplexe die Ringöffnende Metathese ohne photochemische oder chemische Aktivierung initiieren können (X. Sauvage, Y. Borguet, A. F. Noels, L. Delaude, A. Demonceau, Adv. Synth. Catal. 2007, 349, 255-265). Dabei wird eine Mischung aus Cycloisomerisierungs- und RCM-Produkten erhalten, wenn α,ω-Diene in Gegenwart dieser Komplexe umgesetzt werden.

Die Aufgabe der vorliegenden Erfindung ist es daher, ein Verfahren zur Herstellung von Olefinen mittels Metathese bereitzustellen, bei dem der Katalysator aus preiswerten Ruthenium-Verbindungen und dem Olefin "in situ" generiert werden soll, aber ohne die zuvor beschriebenen Aktivierungsmittel auskommt.

Die Aufgabe wird gelöst durch ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}] _{z} (I), wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt.

Im Sinne der vorliegenden Erfindung bezieht sich der Begriff Olefin auf alle Arten von Olefinen unabhängig davon, ob es Mono- oder Diolefine, ob es cyclische Olefine oder acyclische interne Olefine oder acyclische terminale Olefine sind, oder auch Mischungen von Olefinen sind.

Ein acyclisches internes Olefin bezeichnet ein Olefin dessen C-C-Doppelbindung sich nicht am alpha-Kohlenstoff befindet. Ein terminales Olefin bezeichnet ein Olefin dessen C-C-Doppelbindung sich am alpha-Kohlenstoff befindet.

Ein Diolefin bezeichnet ein Olefin mit zwei C-C-Doppelbindungen in einem Molekül, wobei ein terminales Diolefin die C-C-Doppelbindungen am alpha- und omega-Kohlenstoffatom aufweist und ein internes Diolefin die C-C-Doppelbindungen weder am alpha- noch am omega-Kohlenstoffatom aufweist. Die Olefine können auch substituiert sein. Beispiele für Olefine sind die Mono- und Diolefine der allgemeinen Formeln (a)-(g), wobei n unabhängig voneinander 1, 2, 3, 4, 5, 6, 7, 8, 9, 10, 11, 12 sein kann, und 1 bis 4 Kohlenstoffatome durch je ein Heteroatom ausgewählt aus der Gruppe bestehend aus N, O, S, P und Si ersetzt sein können, und die Wasserstoffatome der Reste (CₙH₂ₙ), (CₙH₂ₙ₊₁), NH, PH, POH und SiH₂ substituiert sein können.

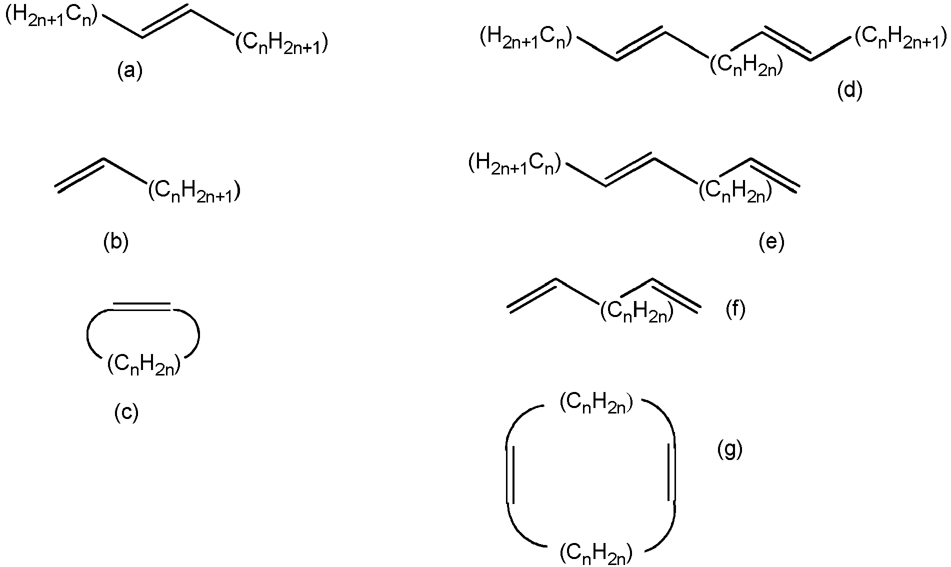

Die Substituenten der Olefine werden ausgewählt aus der Gruppe bestehend aus
- {C₁-C₂₀}-Alkyl,
- {C₃-C₈}-Cycloalkyl,
- {C₃-C₇}-Heterocycloalkyl,
- {C₆-C₁₄}-Aryl,
- {C₃-C₁₄}-Heteroaryl,
- {C₆₋C₁₄}-Aralkyl,
- {C₁-C₂₀}-Alkyloxy,
- {C₆-C₁₄}-Aryloxy,
- {C₆-C₁₄}-Aralkyloxy,
- {C₁-C₂₀}-Alkylthio,
- {C₆-C₁₄}-Arylthio,
- {C₆-C₁₄}-Aralkylthio,
- {C₁-C₈}-Acyl,
- {C₁-C₈}-Acyloxy,
- OH,
- NH2,
- NH({C₁-C₂₀}-Alkyl),
- NH({C₆-C₁₄}-Aryl),
- NH({C₆-C₁₄}-aralkyl),
- NH({C₁-C₈}-Acyl),
- NH({C₁-C₈}-Acyloxy)
- N((C₁-C₂₀)-Alkyl)₂,
- N({C₆-C₁₄}-Aryl)₂,
- N({C₆-C₁₄}-Aralkyl)₂,
- N({C₁-C₂₀}-Alkyl)({C₆-C₁₄}-Aryl),
- N({C₁-C₈}-Acyl)₂,
- NH3⁺,
- NH({C₁-C₂₀}-Alkyl)₂⁺,
- NH({C₆-C₁₄}-Aryl)₂⁺,
- NH({C₆-C₁₄}-Araky)₂⁺,
- NH({C₁-C₂₀}-Alkyl)({C₆-C₁₄}-Aryl)⁺,
- N({C₆-C₁₄}-Aryl)ₓ({C₁-C₂₀}-Alkyl)₃₋ₓ⁺,
- NO₂,
- O-C(=O)-O-{C₁-C₂₀}-Alkyl,
- O-C(=O)-O-{C₆-C₁₄}-Aryl,
- O-C(=O)-O-{C₆-C1₄}-Aralkyl,
- NH-C(=O)-O-{C₁-C₂₀}-Alkyl,
- NH-C(=O)-O-{C₆-C₁₄}-Aryl,
- NH-C(=O)-O-{C₆-C₁₄}-Aralkyl,
- O-C(=O)-NH-{C₁-C₂₀}-Alkyl,
- O-C(=O)-NH-{C₆-C₁₄}-Aryl,
- O-C(=O)-NH-{C₆-C₁₄}-Aralkyl,
- NH-C(=O)-NH₂,
- NH-C(=O)-NH-{C₁-C₂₀}-Alkyl,
- NH-C(=O)-NH-{C₆-C₁₄}-Aryl,
- NH-C(=O)-NH-{C₆-C₁₄}-Aralkyl,
- CN,
- Halogen,
- C(=N-{C₁-C₂₀}-Alkyl)-{C₁-C₂₀}-Alkyl,
- C(=N-{C₆-C₁₄}-Aryl)-{C₁-C₂₀}-Alkyl,
- C(=N-{C₁-C₂₀}-Alkyl)-{C₆-C₁₄}-Aryl,
- C(=N-{C₆-C₁₄}-Aryl)-{C₆-C₁₄}-Aryl,
- SO₂-O-{C₁-C₂₀}-Alkyl,
- SO₂-O-{C₆-C₁₄}-Aryl,
- SO₂-O-{C₆-C₁₄}-Aralkyl,
- SO₂-{C₁-C₂₀}-Alkyl,
- SO₂-{C₆-C₁₄}-Aryl,
- SO₂-{C₆-C₁₄}-Aralkyl,
- SO-{C₁-C₂₀}-Alkyl,
- SO-{C₆-C₁₄}-Aryl,
- SO-{C₆-C₁₄}-Aralkyl,
- Si({C₁-C₂₀}-Alkyl)₃,
- Si({C₆-C₁₄}-Aryl)₃,
- Si({C₆-C₁₄}-Aryl)ₓ({C₁-C₂₀}-Alkyl)₃₋ₓ,
- {C₁-C₂₀}-Perfluoroalkyl,
- PO(O-{C₁-C₂₀}-Alkyl)₂,
- PO(0-{C₆-C₁₄}-Aryl)₂,
- PO(O-{C₁-C₂₀}-Alkyl)(O-{C₆-C₁₄}-Aryl),
- PO({C₁-C₂₀}-Alkyl)₂,
- PO({C₆-C₁₄}-Aryl)₂,
- PO({C₁-C₂₀}-Alkyl)({C₆-C₁₄}-Aryl).

### Beispiele für Mono- und Diolefine sind die folgenden Verbindungen

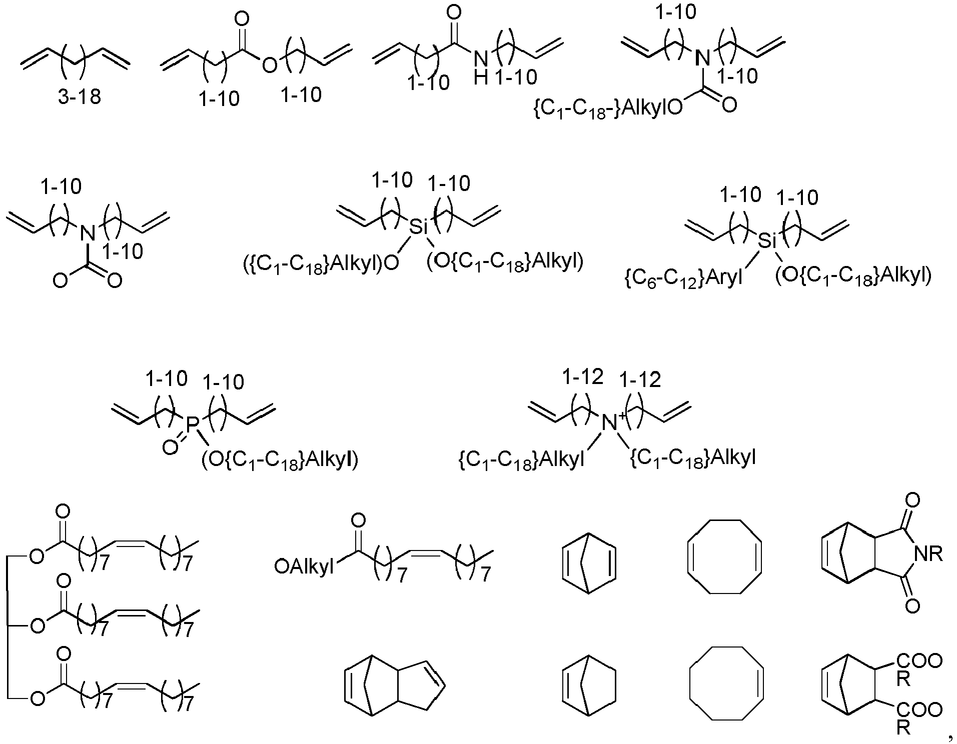

wobei R für (C₁-C₁₈)-Alkyl oder (C₆-C₁₂) -Aryl steht und die Olefine unsubstituiert oder wie zuvor beschrieben substituiert sein können.

Bevorzugt werden terminale Monoolefine oder terminale Diolefine eingesetzt, die unsubstituiert oder wie zuvor beschrieben substituiert sein können.

### Besonders bevorzugt sind die Olefine ausgewählt aus der Gruppe bestehend aus den folgenden Verbindungen, die unsubstituiert oder wie zuvor beschrieben substituiert sein können

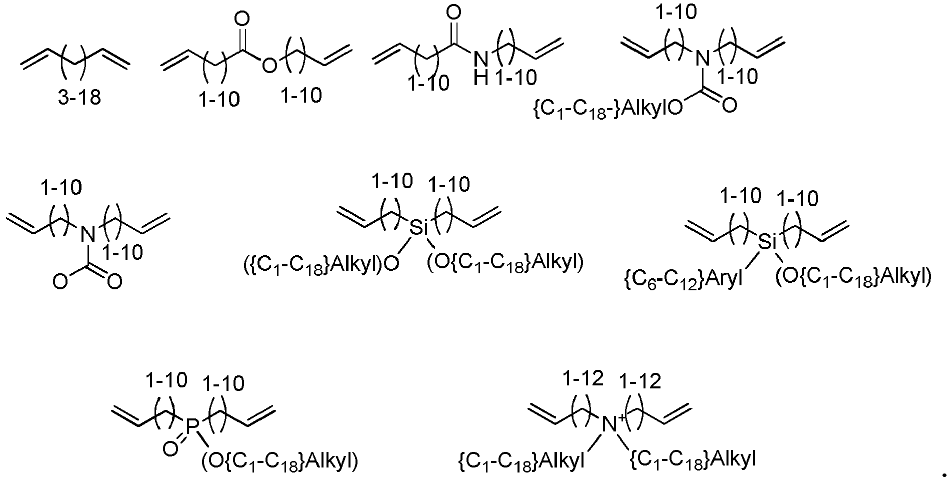

Im Sinne der vorliegenden Erfindung bezeichnen anionische Liganden einfach oder mehrfach negativ geladene Liganden, die unabhängig voneinander ausgewählt werden aus der Gruppe bestehend aus Halogenid, Pseudohalogenid, Tetraphenylborat, Hexahalogenphosphat, Methansulfonat, Trihalogenomethansulfonat, Arylsulfonat, Alkoxid, Aryloxid, Carboxylat, Sulfat oder Phosphat. Pseudohalogenide sind Liganden, die sich chemisch ähnlich wie die Halogenide verhalten, wobei unter den Pseudohalogeniden Cyanid (CN⁻), Cyanat (OCN⁻), Thiocyanat oder Rhodanid (SCN⁻) bevorzugt sind. Bevorzugt sind anionische Liganden unabhängig voneinander ausgewählt aus der Gruppe bestehend aus den Halogeniden Fluorid, Chlorid, Bromid und Iodid, wobei Chlorid besonders bevorzugt ist.

Unter einem neutralen π-bindenden Liganden werden monocyclische und polycyclische Arene verstanden, die auch Substituenten aufweisen können, welche identisch oder nichtidentisch sein können, verstanden, wobei die Substituenten ausgewählt werden aus der Gruppe bestehend aus (C₁-C₂₀) -Alkyl-, (C₆-C₁₄) -Aryl-, (C₁-C₂₀)-Alkyloxy-, (C₆-C₁₄)-Aryloxy-, (C₁-C₂₀)-Perfluoroalkyl-, (C₁-C₂₀)-Alkylthio-, (C₂-C₁₀)-Alkenylthio-, (C₂-C₁₀)-Alkenyl-, (C₂-C₁₀)-Alkinyl-, (C₂-C₁₀)-Alkenyloxy-, (C₂-C₁₀)-Alkinyloxy-, und Halogen. Die Substituenten können ihrerseits ebenfalls substituiert sein, wobei diese Substituenten ausgewählt werden aus der Gruppe bestehend aus Halogen, (C₁-C₈) -Alkyl, (C₁-C₈) - Alkyloxy, -NH₂, -NO, -NO₂, NH (C₁-C₈) -Alkyl, -N((C₁-C₈)-Alkyl)₂, -OH, -CF₃, -CₙF₂ₙ₊₁ (wobei n eine ganze Zahl von 2 bis 5 ist), NH(C₁-C₈)-Acyl, -N((C₁-C₈)-Acyl)₂, (C₁-C₈)-Acyl, (C₁-C₈)-Acyloxy, -SO₂-(C₁-C₈)-Alkyl, -SO₂-(C₆-C₁₄)-Aryl, -SO-(C₁-C₈)-Alkyl, -SO-(C₆-C₁₄)-Aryl, -PO(O-{C₁-C₂₀}-Alkyl)₂, -PO(O-{C₆-C₁₄}-Aryl)₂, - PO(O-{C₁-C₂₀}-Alkyl) (O-{C₆-C₁₄}-Aryl), -PO({C₁-C₂₀}-Alkyl)₂, - PO({C₆-C₁₄}-Aryl)₂, -PO({C₁-C₂₀}-Alkyl) ({C₆-C₁₄}-Aryl). Beispiele sind Benzol, Toluol, Xylol, Cymen, Trimethylbenzol, Tetramethylbenzol, Hexamethylbenzol, Tetrahydronaphthalin, und Naphthalin. Besonders bevorzugt wird der neutrale π-bindende Ligand ausgewählt aus der Gruppe bestehend aus Benzol, Cymen und Hexamethylbenzol.

Unter einem neutralen Elektronendonor-Liganden wird ein Ligand ohne Nettoladung verstanden, der Freie Elektronenpaare oder mit Elektronen-gefüllte Orbitale für eine koordinative Bindung mit einem Akzeptor zur Verfügung stellt. Ein Akzeptor ist ein Atom, das freie Elektronen oder Elektronen aus einem gefüllten Orbital vom Donor aufnehmen kann. Donoren sind typischerweise Hauptgruppenelemente der Gruppen 13-17 des Periodensystems der Elemente, wie z.B. C, N, P. Selbst Kohlenstoff kann als neutraler Elektronendonor auftreten. Am häufigsten tritt Kohlenstoff in Carbenen als neutraler Elektronendonor auf, wobei das Kohlenstoffatom ein Elektronenpaar in einem Orbital trägt. Diese Elektronen stehen für eine sigma-Bindung mit einem Akzeptoratom zur Verfügung. Akzeptoren sind typischerweise Metallatome, wie z.B. Pd(0), Pd(II), Ru (I) oder Ru(II).

Typische Beispiele für neutrale Elektronendonor-Liganden sind heterocyclische Carbene, Phosphane, Phosphinite, Phosphonite, Phosphite, Arsane und Stickstoffbasen. Besonders bevorzugt ist der neutrale Elektronendonor-Ligand ausgewählt aus der Gruppe bestehend aus Phosphanen und N-heterocyclischen Carbenen. Bevorzugt sind N-heterocyclische Carbene ausgewählt aus der Gruppe bestehend aus Verbindungen der Formeln VI - XI und Phosphane ausgewählt aus der Gruppe bestehend aus P(Phenyl)₃ und P(Cyclohexyl)₃.

Im Sinne der vorliegenden Erfindung wird unter einem heterocyclischen Carben ein Carben der allgemeinen Formel (V) verstanden 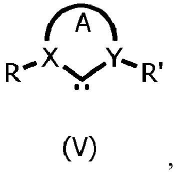 wobei
R und R' gleich oder verschieden sind und ausgewählt werden aus der Gruppe bestehend aus
Wasserstoff,
(C₁-C₁₈)-Alkyl,
(C₃-C₈)-Cycloalkyl,
(C₃-C₇)-Heterocycloalkyl,
(C₆-C₁₄) -Aryl,
und (C₃-C₁₄) -Heteroaryl;
X und Y unabhängig voneinander ausgewählt werden aus der Gruppe bestehend aus Kohlenstoff-, Stickstoff- und Phosphoratom, und
A eine (C₂-C₄) -Alkylenbrücke oder eine (C₂-C₄) - Heteroalkylenbrücke ist.

Beispiele für heterocyclische Carbene sind die N-hetercyclischen Carbene der Formeln VI bis XI:

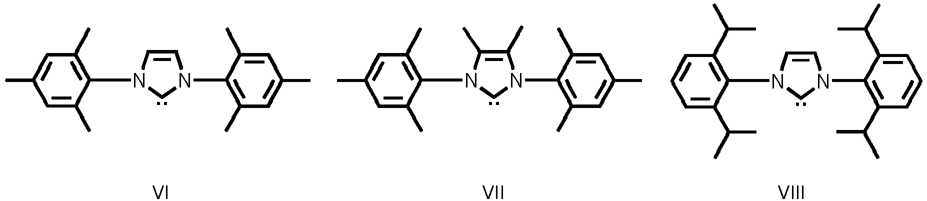

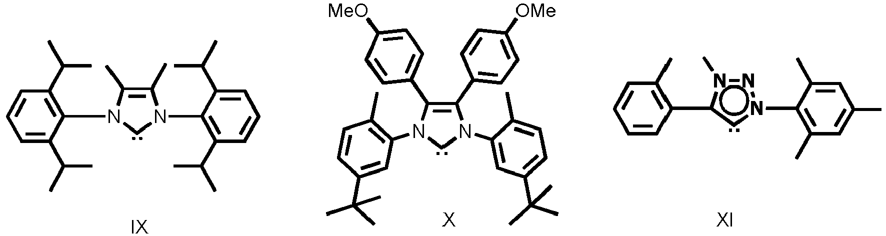

N-heterocyclische Carbene können in situ erhalten werden durch thermische Aktivierung von Carben-Addukten oder die Kombination einer N-heterocyclischen Carben-Vorstufe und einer Base. Als Base kann dabei jede anorganische und organische Base eingesetzt werden, bevorzugt werden Stickstoffbasen oder Alkyloxybase eingesetzt. Besonders bevorzugt sind Alkyloxybasen, die ausgewählt werden aus der Gruppe enthaltend Natrium- oder Kaliumsalze von Methanolat, Ethanolat, Propanolat oder Butanolat und deren Isomere. Typische Beispiele für Carben-Addukte sind Addukte von N-heterocyclischen Carbenen mit Alkoholen, Chloroform, Pentafluorophenol, CO₂ und Boran.

Im Sinne der vorliegenden Erfindung bezeichnen Phosphane Verbindungen der allgemeinen Formel PR¹R²R³, in der R¹, R² und R³ gleich oder verschieden, ausgewählt aus der Gruppe H, (C₁-C₁₈) -Alkyl, (C₃-C₈) -Cycloalkyl-, (C₂-C₇)-Heterocycloalkyl-, (C₆-C₁₄) -Aryl- oder (C₃-C₁₄) -Heteroaryl, sein können, wobei gegebenenfalls die Reste R¹, R² und R³ ein oder mehrere cyclische Strukturen aufweisen können. Typische Beispiele sind P(Phenyl)₃, P(Cyclohexyl)₃, P(Isopropyl))₃, P(Cyclopentyl)₃, P(Tertbutyl)₃, P (Neopentyl) ₃, wobei P(Phenyl)₃, P(Cyclohexyl)₃ und P(Isopropyl)₃ besonders bevorzugt sind.

Im Sinne der vorliegenden Erfindung bezeichnen Phosphinite Verbindungen der allgemeinen Formel PR^{1'}R^{2'}(OR^{3'}), in der R^{1'} und R^{2'} gleich oder verschieden, ausgewählt aus der Gruppe (C₁-C₁₈) -Alkyl, (C₃-C₈) -Cycloalkyl-, (C₂-C₇)-Heterocycloalkyl-, (C₆-C₁₄) -Aryl- oder (C₃-C₁₄) -Heteroaryl, und R^{3'} ausgewählt aus der Gruppe H, (C₁-C₈)-Alkyl, (C₆-C₁₄) -Aryl sein können, wobei gegebenenfalls die Reste R^{1'}, R^{2'} und R^{3'} ein oder mehrere cyclische Strukturen aufweisen können. Typische Beispiele sind (Tertbutyl)₂P(OButyl), (1-Adamantyl)₂P(OButyl), Ph₂P(OEt), Ph₂P(OPh), P (OPh) ₃, Ph₂P(O-(2,4-ditertbutyl)phenyl).

Im Sinne der vorliegenden Erfindung bezeichnen Phosphonite Verbindungen der allgemeinen Formel P(OR^{1"})(OR^{2"})R^{3"}, in der R^{1"} und R^{2"} gleich oder verschieden, ausgewählt aus der Gruppe (C₁-C₈) -Alkyl, (C₆-C₁₄) -Aryl, und R^{3"} ausgewählt aus der Gruppe H, (C₁-C₁₈) -Alkyl, (C₃-C₈) -Cycloalkyl-, (C₂-C₇) - Heterocycloalkyl-, (C₆-C₁₄)-Aryl- oder (C₃-C₁₄) -Heteroaryl sein können, wobei gegebenenfalls die Reste R^{1"}, R^{2"} und R^{3"} ein oder mehrere cyclische Strukturen aufweisen können. Typische Beispiele sind MeP(OMe)₂, EtP(OEt)₂, PhP(OEt)₂, PhP(OPh)₂, PhCH₂P(OPh)₃ und PhP(O-(2,4-ditertbutyl)phenyl)₂.

Im Sinne der vorliegenden Erfindung bezeichnen Phosphite Verbindungen der allgemeinen Formel P(OR^{1"'})(OR^{2"'})(OR^{3"'}), in der R^{1"'}, R^{2"'} und R^{3"'} gleich oder verschieden, ausgewählt aus der Gruppe H, (C₁-C₈) -Alkyl, (C₆-C₁₄)-Aryl sein können, wobei gegebenenfalls die Reste R^{1"'}, R^{2"'} und R^{3"'} ein oder mehrere cyclische Strukturen aufweisen können. Typische Beispiele sind P(OMethyl)₃, P(OEthyl)₃, P(OPhenyl)₃, P(O-(2,4-ditertbutyl)phenyl)₃ und

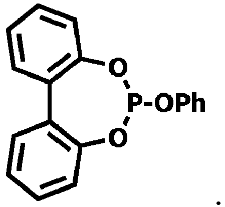

Im Sinne der vorliegenden Erfindung bezeichnen Arsane Verbindungen der allgemeinen Formel AsR^{1""}R^{2""}R^{3""}, in der R^{1""} , R^{2""} und R^{3""} gleich oder verschieden, ausgewählt aus der Gruppe H, (C₁-C₁₈)-Alkyl, (C₃-C₈)-Cycloalkyl-, (C₂-C₇)-Heterocycloalkyl-, (C₆-C₁₄)-Aryl- oder (C₃-C₁₄)-Heteroaryl, sein können, wobei gegebenenfalls die Reste R¹, R² und R³ ein oder mehrere cyclische Strukturen aufweisen können. Typische Beispiele sind As(Phenyl)₃, As(Cyclohexyl)₃, As(Isopropyl)₃, As(Cyclopentyl)₃, As(Tertbutyl)₃, As(Neopentyl)₃, wobei As(Phenyl)₃, As(Cyclohexyl)₃ und As(Isopropyl)₃ besonders bevorzugt sind.

Im Sinne der vorliegenden Erfindung bezeichnen Stickstoffbasen Amine und Stickstoffaromaten. Beispiele für Amine sind Ammoniak, Triethylamin, N,N-Dimethylanilin, Piperidin, N-Methylpyrrolidin, 1,4-Diazabicyclo[2.2.2]octan oder 1,8-Diazobicyclo[5.4.0]undec-7-en, wobei Triethylamin besonders bevorzugt ist. Beispiele für Stickstoffaromaten sind Pyridin, Pyrimidin, Pyrazin, Pyrrol, Indol, Carbazol, Imidazol, Pyrazol, Benzimidazol, Oxazol, Thiazol, Isoxazol, Isothiazol, Triazol, Chinolin, Isochinolin, Acridin, Phenazin, Phenoxazin, Phenothiazin oder Triazin, wobei Pyridin besonders bevorzugt ist.

Im Sinne der vorliegenden Erfindung ist (C₁-Cₙ)-Alkyl definiert als lineare oder verzweigte C₁-Cₙ-Alkylgruppe mit 1 bis n Kohlenstoffatomen. Typische Beispiele für C₁-Cₙ-Alkylgruppen sind Methyl, Ethyl, n-Propyl, Isopropyl, n-Butyl, Isobytyl, sec-Butyl, tert-Butyl, Pentyl, Hexyl, Heptyl, Octyl, Nonanyl, Decanyl, Dodecanyl, oder Octadecanyl samt aller ihrer Bindungsisomeren. Eine (C₁-Cₙ)-Alkylgruppe kann auch mit wenigstens einem Substituenten substitutiert sein, wobei die Substituenten unabhängig voneinander ausgewählt werden aus der Gruppe bestehend aus Halogen, (C₁-C₈) -Alkyl, (C₁-C₈) -Alkyloxy, - NH₂, -NO, -NO₂, NH(C₁-C₈)-Alkyl, -N((C₁-C₈)-Alkyl)₂, -OH, -CF₃,-CₙF₂ₙ₊₁ (wobei n eine ganze Zahl von 2 bis 5 ist), NH(C₁-C₈)-Acyl, -N((C₁-C₈)-Acyl)₂, (C₁-C₈)-Acyl, (C₁-C₈)-Acyloxy, -SO₂-(C₁-C₈)-Alkyl, -SO₂- (C₆-C₁₄)-Aryl, -SO-(C₁-C₈)-Alkyl, -SO-(C₆-C₁₄)-Aryl, -PO(O-{C₁-C₂₀}-Alkyl)₂, -PO(O-{C₆-C₁₄}-Aryl)₂, -PO(O-{C₁-C₂₀}-Alkyl)(O-{C₆-C₁₄}-Aryl), -PO({C₁-C₂₀}-Alkyl)₂, -PO({C₆-C₁₄}-Aryl)₂, -PO({C₁-C₂₀}-Alkyl)({C₆-C₁₄}-Aryl).

Im Sinne der vorliegenden Erfindung bezeichnet (C₃-Cₙ)-Cycloalkyl eine cyclische Alkylgruppe mit 3 bis n Kohlenstoffatomen, wobei mono-, bi- und tricyclische Alkylgruppen umfasst sind. Typische Beispiele sind Cyclopropyl, Cyclobutyl, Cyclopentyl, Cyclohexyl und Cycloheptyl. Eine (C₁-Cₙ)-Cycloalkylgruppe kann auch mit wenigstens einem Substituenten substitutiert sein, wobei die Substituenten unabhängig voneinander ausgewählt werden aus der Gruppe bestehend aus Halogen, (C₁-C₈)-Alkyl, (C₁-C₈)-Alkyloxy, - NH₂, -NO, -NO₂, NH(C₁-C₈)-Alkyl, -N((C₁-C₈)-Alkyl)₂, -OH, -CF₃, -CₙF₂ₙ₊₁ (wobei n eine ganze Zahl von 2 bis 5 ist), NH(C₁-C₈)-Acyl, -N((C₁-C₈)-Acyl)₂, (C₁-C₈)-Acyl, (C₁-C₈)-Acyloxy, -SO₂-(C₁-C₈)-Alkyl, -SO₂-(C₆-C₁₄)-Aryl, -SO-(C₁-C₈)-Alkyl, -SO-(C₆-C₁₄)-Aryl, -PO(O-{C₁-C₂₀}-Alkyl)₂, -PO(O-{C₆-C₁₄}-Aryl)₂, -PO(O-{C₁-C₂₀}-Alkyl)(O-{C₆-C₁₄}-Aryl), -PO({C₁-C₂₀}-Alkyl)₂, -PO({C₆-C₁₄}-Aryl)₂, -PO({C₁-C₂₀}-Alkyl) ({C₆-C₁₄}-Aryl).

Im Sinne der vorliegenden Erfindung bezeichnet (C₂-Cₙ)-Heterocycloalkyl eine cyclische Alkylgruppe mit 2 bis n Kohlenstoffatomen, wobei mono-, bi- und tricyclische Alkylgruppen umfasst sind, worin 1 oder 2 Kohlenstoffatome des Zyklus durch je ein Heteroatom ausgewählt aus der Gruppe bestehend aus N, O und S ersetzt sind. Eine (C₂-Cₙ)-Heterocycloalkylgruppe kann auch mit wenigstens einem Substituenten substitutiert sein, wobei die Substituenten unabhängig voneinander ausgewählt werden aus der Gruppe bestehend aus Halogen, (C₁-C₈)-Alkyl, (C₁-C₈)-Alkyloxy, -NH₂, - NO, -NO₂, NH(C₁-C₈))-Alkyl, - N((C₁-C₈)-Alkyl)₂, -OH, -CF₃, - CₙF₂ₙ₊₁ (wobei n eine ganze Zahl von 2 bis 5 ist), NH(C₁-C₈)-Acyl, -N((C₁-C₈)-Acyl)₂, (C₁-C₈)-Acyl, (C₁-C₈)-Acyloxy, -SO₂-(C₁-C₈) -Alkyl, -SO₂-(C₆-C₁₄)-Aryl, -SO-(C₁-C₈)-Alkyl, -SO-(C₆-C₁₄)-Aryl, -PO(O-{C₁-C₂₀}-Alkyl)₂, -PO(O-{C₆-C₁₄}-Aryl)₂, -PO(O-{C₁-C₂₀}-Alkyl)(O-{C₆-C₁₄}-Aryl), -PO({C₁-C₂₀}-Alkyl)₂, -PO({C₆-C₁₄}-Aryl)₂, -PO({C₁-C₂₀}-Alkyl)({C₆-C₁₄}-Aryl). Typische Beispiele sind 2- oder 3-Tetrahydrofuryl, 1-, 2- oder 3-Pyrrolidinyl, 1-, 2-, 3- oder 4-Piperidinyl, 1-, 2-, oder 3-Morpholinyl, 1-, oder 2-Piperazinyl, 1-Caprolactyl.

Im Sinne der vorliegenden Erfindung ist (C₆-Cₙ)-Aryl eine cyclische aromatische Gruppe mit 6 bis n Kohlenstoffatomen. Insbesondere zählen hierzu Verbindungen wie Phenyl-, Naphthyl-, Anthryl-, Phenanthryl-, Biphenylreste oder an das betreffende Molekül annelierte Systeme der vorbeschriebenen Art, wie z.B. Indenylsysteme. Eine (C₆-Cₙ)-Arylgruppe kann auch mit wenigstens einem Substituenten substitutiert sein, wobei die Substituenten unabhängig voneinander ausgewählt werden aus der Gruppe bestehend aus Halogen, (C₁-C₈) -Alkyl, (C₁-C₈) - Alkyloxy, -NH₂, -NO, -NO₂, NH(C₁-C₈))-Alkyl, - N((C₁-C₈)-Alkyl)₂, -OH, -CF₃, -CₙF₂ₙ₊₁ (wobei n eine ganze Zahl von 2 bis 5 ist), NH(C₁-C₈)-Acyl, -N((C₁-C₈)-Acyl)₂, (C₁-C₈)-Acyl, (C₁-C₈)-Acyloxy, -SO₂-(C₁-C₈)-Alkyl, -SO₂-(C₆-C₁₄)-Aryl, -SO-(C₁-C₈)-Alkyl, -SO-(C₆-C₁₄)-Aryl, -PO(O-{C₁-C₂₀}-Alkyl)₂, -PO(O-{C₆-C₁₄}-Aryl)₂, - PO(O-{C₁-C₂₀}-Alkyl) (O-{C₆-C₁₄}-Aryl), -PO({C₁-C₂₀}-Alkyl)₂, - PO({C₆-C₁₄}-Aryl)₂, -PO({C₁-C₂₀}-Alkyl) ({C₆-C₁₄}-Aryl).

Im Sinne der vorliegenden Erfindung bezeichnet (C₃-Cₙ)-Heteroaryl ein fünf-, sechs- oder siebengliedriges aromatisches Ringsystem aus 3 bis n C-Atomen, wobei 1 bis 3 Kohlenstoffatome des Ringssystems durch je ein Heteroatom ausgewählt aus der Gruppe bestehend aus N, O und S ersetzt sind. Als solche Heteroarylgruppen werden insbesondere Gruppen angesehen, wie 2-, 3-Furyl, 2-, 3-Pyrrolyl, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl. Eine (C₃-Cₙ)-Heteroarylgruppe kann auch mit wenigstens einem Substituenten substitutiert sein, wobei die Substituenten unabhängig voneinander ausgewählt werden aus der Gruppe bestehend aus Halogen, (C₁-C₈) -Alkyl, (C₁-C₈) -Alkyloxy, -NH₂, -NO, -NO₂, NH (C₁-C₈) -Alkyl, - N((C₁-C₈)-Alkyl)₂, -OH, -CF₃, -CₙF₂ₙ₊₁ (wobei n eine ganze Zahl von 2 bis 5 ist), NH(C₁-C₈)-Acyl, -N((C₁-C₈)-Acyl)₂, (C₁-C₈) -Acyl, (C₁-C₈)-Acyloxy, -SO₂-(C₁-C₈)-Alkyl, -SO₂-(C₆-C₁₄)-Aryl, -SO-(C₁-C₈)-Alkyl, -SO-(C₆-C₁₄)-Aryl, -PO(O-{C₁-C₂₀}-Alkyl)₂, -PO(O-{C₆-C₁₄}-Aryl)₂, -PO(O-{C₁-C₂₀}-Alkyl)(O-{C₆-C₁₄}-Aryl), -PO({C₁-C₂₀}-Alkyl)₂, -PO({C₆-C₁₄}-Aryl)₂, -PO({C₁-C₂₀}-Alkyl) ({C₆-C₁₄}-Aryl).

Im Sinne der vorliegenden Erfindung bezeichnet (C₆-Cₙ)-Aralkyl eine Gruppe, die sowohl eine Alkyl- als auch eine Arylgruppe enthält und in Summe 6 bis n Kohlenstoffatome aufweist. Die Aralkylgruppe kann über jedes ihrer Kohlenstoffatome an das diese Gruppe tragende Molekül gebunden sein. Eine (C₆-Cₙ)-Aralkylgruppe kann auch mit wenigstens einem Substituenten substitutiert sein, wobei die Substituenten unabhängig voneinander ausgewählt werden aus der Gruppe bestehend aus Halogen, (C₁-C₈) -Alkyl, (C₁-C₈) -Alkyloxy, -NH₂, -NO, -NO₂, NH(C₁-C₈) -Alkyl, - N((C₁-C₈)-Alkyl)₂, -OH, -CF₃, -CₙF₂ₙ₊₁ (wobei n eine ganze Zahl von 2 bis 5 ist), NH (C₁-C₈) -Acyl, -N((C₁-C₈)-Acyl)₂, (C₁-C₈) -Acyl, (C₁-C₈) -Acyloxy, -SO₂- (C₁-C₈) -Alkyl, -SO₂- (C₆-C₁₄) - Aryl, -SO- (C₁-C₈) -Alkyl, -SO- (C₆-C₁₄) -Aryl, -PO(O-{C₁-C₂₀}-Alkyl)₂, -PO(O-{C₆-C₁₄}-Aryl)₂, -PO(O-{C₁-C₂₀}-Alkyl)(O-{C₆-C₁₄}-Aryl), -PO({C₁-C₂₀}-Alkyl)₂, -PO({C₆-C₁₄}-Aryl)₂, -PO({C₁-C₂₀}-Alkyl) ({C₆-C₁₄}-Aryl).

Im Sinne der vorliegenden Erfindung bezeichnet (C₂-Cₙ)-Alkylen eine divalente lineare (C₂-Cₙ)-Alkylgruppe mit 2 bis n Kohlenstoffatomen. Typische Beispiele sind Ethylen, n-Propylen, Isopropylen, n-Butylen, Isobutylen, tert-Butylen, n-Pentylen, n-Hexylen, n-Heptylen, n-Octylen, n-Nonylen, n-Decylen. Die (C₂-Cₙ)-Alkylengruppe kann auch Substituenten aufweisen, die ausgewählt werden aus der Gruppe bestehend aus (C₁-C₂₀)-Alkyl, (C₆-C₁₄)-Aryl, (C₂-C₁₀)-Alkenyl. Außerdem kann die (C₂-Cₙ)-Alkylengruppe ungesättigt sein, wobei der ungesättigte Abschnitt Teil eines aromatischen oder heteroaromatischen Systems sein kann.

Im Sinne der vorliegenden Erfindung bezeichnet (C₂-Cₙ)-Heteroalkylen eine divalente lineare (C₂-Cₙ)-Alkylgruppe mit 2 bis n Kohlenstoffatomen, wobei 1 oder 2 Kohlenstoffatome durch Heteroatome wie N, O, S ersetzt sind. Die (C₂-Cₙ)-Heteroalkylengruppe kann auch Substituenten aufweisen, die ausgewählt werden aus der Gruppe bestehend aus (C₁-C₂₀)-Alkyl, (C₆-C₁₄) -Aryl, (C₂-C₁₀) -Alkenyl. Außerdem kann die (C₂-Cₙ)-Heteroalkylengruppe ungesättigt sein, wobei der ungesättigte Abschnitt Teil eines aromatischen oder heteroaromatischen Systems sein kann.

Im Sinne der vorliegenden Erfindung ist (C₂-Cₙ)-Alkenyl definiert als lineare oder verzweigte (C₂-Cₙ)-Alkylgruppe mit 2 bis n Kohlenstoffatomen mit der Maßgabe, dass diese eine C-C-Doppelbindung aufweist.

Im Sinne der vorliegenden Erfindung ist (C₂-Cₙ)-Alkinyl definiert als lineare oder verzweigte (C₂-Cₙ)-Alkylgruppe mit 2 bis n Kohlenstoffatomen mit der Maßgabe, dass diese eine C-C-Dreifachbindung aufweist.

Im Sinne der vorliegenden Erfindung ist (C₁-Cₙ)-Alkyloxy definiert als lineare oder verzweigte C₁-Cₙ-Alkylgruppe mit 1 bis n Kohlenstoffatomen mit der Maßgabe, dass diese über ein Sauerstoffatom an das diese Gruppe tragende Molekül gebunden ist.

Im Sinne der vorliegenden Erfindung ist (C₂-Cₙ)-Alkenyloxy definiert als lineare oder verzweigte C₂-Cₙ-Alkenylgruppe mit 2 bis n Kohlenstoffatomen mit der Maßgabe, dass diese über ein Sauerstoffatom an das diese Gruppe tragende Molekül gebunden ist.

Im Sinne der vorliegenden Erfindung ist (C₂-Cₙ)-Alkinyloxy definiert als lineare oder verzweigte C₂-Cₙ-Alkinylgruppe mit 2 bis n Kohlenstoffatomen mit der Maßgabe, dass diese über ein Sauerstoffatom an das diese Gruppe tragende Molekül gebunden ist.

Im Sinne der vorliegenden Erfindung bezeichnet (C₁-Cₙ)-Acyl eine Gruppe mit der allgemeinen Struktur R-(C=O)- mit insgesamt 1 bis n Kohlenstoffatomen, wobei R ausgewählt wird aus der Gruppe bestehend aus H, (C₁-Cₙ₋₁)-Alkyl, (C₁-Cₙ₋₁)-Alkenyl, (C₆-Cₙ₋₁) -Aryl, (C₆-Cₙ₋₁) -Heteroaryl und (C₂-C_{n-d}-Alkinyl.

Im Sinne der vorliegenden Erfindung bezeichnet (C₁-Cₙ)-Acyloxy eine Gruppe mit der allgemeinen Struktur R-(C=O)O- mit insgesamt 1 bis n Kohlenstoffatomen, wobei R ausgewählt wird aus der Gruppe bestehend aus H, (C₁-Cₙ₋₁)-Alkyl, (C₁-Cₙ₋₁)-Alkenyl, (C₆-Cₙ₋₁) -Aryl, (C₆-Cₙ₋₁) -Heteroaryl und (C₂-Cₙ₋₁-Alkinyl.

Im Sinne der vorliegenden Erfindung handelt es sich bei einem nicht-koordinierenden Salz um ein anorganisches Salz, das ausgewählt wird aus der Gruppe bestehend aus einem Natrium-, Kalium, Caesium-, Barium-, Calcium- und Magnesium-Salz von PF₆⁻, BF₄⁻, BH₄⁻, F₃CSO₃, H₃CSO₃⁻, ClO₄⁻, SO₄²⁻, HSO₄⁻, NO₃⁻, PO4³⁻, HPO₄²-, H₂PO₄⁻, CF₃COO⁻, B(C₆F₅)₄⁻, B[3,5-(CF₃)₂C₆H_{3]4}⁻, RSO₃⁻, und R'COO⁻, wobei R, R' unabhängig voneinander ausgewählt sind aus der Gruppe bestehend aus (C₁-C₂₀)-Alkyl und (C₆-C₁₄)-Aryl. Bevorzugt wird R ausgewählt aus der Gruppe bestehend aus Methyl, Phenyl, p-Tolyl, und p-Nitrophenyl, R' wird bevorzugt ausgewählt aus der Gruppe bestehend aus H, Methyl, Phenyl, p-Tolyl, und p-Nitrophenyl. Bevorzugt wird das nicht-koordinierende Salz ausgewählt aus der Gruppe bestehend aus einem Natrium-, Kalium, Caesium-, Barium-, Calcium- und Magnesium-Salz von PF₆⁻, BF₄⁻, F₃CSO₃⁻, CF₃COO⁻, B (C₆F₅) ₄⁻, besonders bevorzugt ist das nicht-koordinierende Salz NaPF₆ oder KPF₆.

Im Sinne der vorliegenden Erfindung bezeichnet eine Lewissäure eine Verbindung, die ausgewählt wird aus der Gruppe bestehend aus Aluminium-, Bor-, Chrom-, Kobalt-, Eisen-, Kupfer-, Magnesium-, Lanthan-, Mangan-, Nickel-, Palladium- oder Zink-Salzen von Cl-, Br-, I-, PF₆-, BF₄-, CF₃COO-, B (C₆F₅) ₄-, B [3, 5-(CF₃)₂C₆H₃]₄-, R^{x}COO-, (R^{y}COCHCOR^{z})-, wobei R^{x}, R^{y}, R^{z} unabhängig voneinander ausgewählt sind aus der Gruppe bestehend aus (C₁-C₂₀) -Alkyl und (C₆-C₁₄) -Aryl . Bevorzugt werden R^{x}, R^{y}, R^{z} unabhängig voneinander ausgewählt sind aus der Gruppe bestehend aus Methyl und Phenyl. Besonders bevorzugt wird die Lewissäure ausgewählt aus der Gruppe bestehend aus Aluminium- oder Eisen-Salzen von Cl-, MeCOO-, oder (MeCOCHCOMe)-, wobei Aluminium(III)chlorid, Eisen (II)acetat und Eisen(III)acetylacetonat besonders bevorzugt sind.

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei in Schritt c) zusätzlich eine Halogenverbindung zugegeben wird.

Im Sinne der vorliegenden Erfindung handelt es sich bei einer Halogenverbindung um eine Verbindung, die ausgewählt wird aus der Gruppe bestehend aus salzartigen Halogeniden und organischen Halogenverbindungen. Salzartige Halogenide werden ausgewählt aus der Gruppe bestehend aus Lithium-, Natrium-, Kalium-, Caesium-, Magnesium-, Calcium- oder AmmoniumHalogeniden, wobei es sich bei Ammoniumhalogeniden um Verbindungen der allgemeinen Formel [NHₓ{(C₁-C₂₀)-Alkyl)₄-ₓ]⁺[Halogenid]⁻ handelt und die Halogenide ausgewählt werden aus der Gruppe bestehend aus Chlorid, Bromid und Iodid. Bei einer organischen Halogenverbindung handelt es sich um eine mono- oder dihalogenierte (C₁-C₂₀)-Alkyl-, (C₃-C₁₀)-Cycloalkyl-, (C₆-C₁₄)-Aryl-, oder (C₁-C₂₀)-Aralkylverbindung, wobei die Halogene unabhängig voneinander ausgewählt werden aus der Gruppe Chlor, Brom oder Iod. Bevorzugt wird die organische Halogenverbindung ausgewählt aus der Gruppe bestehend aus Mono- und Dibromverbindungen, aromatischen 1,2-Organodihalogeniden, und allylischen Monobromiden. Bevorzugt wird das salzartige Halogenid ausgewählt aus der Gruppe bestehend aus Natriumbromid, Natriumiodid, Kaliumchlorid, Kaliumbromid, Kaliumiodid und Tetraalkylammoniumbromiden. Bevorzugt wird die Halogenverbindung ausgewählt aus der Gruppe bestehend aus Kaliumiodid, Kaliumbromid, Tetrabutylammoniumbromid, 1,2-Dibromocyclohexane, 1,2-Bromocyclohexane, 1,2-Dibromoethane, 1,2-Dibromo-4,5-dimethylbenzol, 1,2-Diiodobenzol, 1-Bromo-2-iodo-benzol, 2-Bromo-styrol, (2-Bromoethyl)benzol, und (3-Bromopropyl)benzol.

Die Olefin-Metathese-Reaktionen werden üblicherweise durch das Zusammenbringen eines Olefins oder einer Olefinmischung mit einer Ruthenium-Verbindung der allgemeinen Formel (I), Zugabe einer Lewissäure oder Zugabe einer Lewissäure und eines anionischen, nicht-koordinierenden Salzes, und gegebenenfalls Zugabe einer Halogenverbindung und anschließendem Heizen der Reaktionsmischung bis zur Vollendung der Reaktion durchgeführt, so dass die katalytisch aktive Ruthenium-Verbindung "in situ" gebildet wird. Die Reaktionstemperatur liegt dabei in einem Bereich von 30 °C bis 140 °C, bevorzugt in einem Bereich von 40 °C bis 140 °C, mehr bevorzugt in einem Bereich von 60 °C bis 100 °C, und besonders bevorzugt in einem Bereich von 70 °C bis 100 °C.

Die Reaktionszeit ist nicht kritisch und liegt in einem Bereich von wenigen Minuten bis einigen Stunden, bevorzugt liegt sie in einem Bereich von 30 Minuten bis 3 Stunden.

Die Reaktionen werden im Allgemeinen unter Schutzgasatmosphäre durchgeführt, bevorzugt unter Stickstoff oder Argon, wobei die Anwesenheit von Sauerstoff unter bestimmten Umständen toleriert werden kann. Die Reaktionen können unter bestimmten Umständen in Anwesenheit von Wasser durchgeführt werden.

Die Metathesereaktionen können in allen Lösungsmitteln oder Lösungsmittelmischungen durchgeführt werden, die den Katalysator nicht deaktivieren. Bevorzugt werden aprotische Lösungsmittel mit geringen Koordinationseigenschaften gewählt. Wenn das Olefin flüssig ist, kann die Reaktion ohne Lösungsmittel durchgeführt werden. Lösungsmittel werden bevorzugt aus der Gruppe bestehend aus Dichlormethan, 1,2-Dichlorethan, Benzol, Toluol, Xylol, Halobenzol und Hexan ausgewählt.

Bei Metathesereaktionen ist das Verhältnis von Ruthenium-Verbindung zu Olefin nicht kritisch und liegt im Bereich von 1:10 bis 1:1.000.000. Für die Ringschlussmetathesereaktion liegt das Verhältnis von Ruthenium-Verbindung zu Olefin bevorzugt im Bereich von 1:100 bis 1:10.000. Für die Kreuzmetathesereaktion liegt das Verhältnis von Ruthenium-Verbindung zu Olefin bevorzugt in einem Bereich von 1:10 bis 1:100 und für die Ringöffnende Polymerisationsmetathesereaktion liegt das Verhältnis von Ruthenium-Verbindung zu Olefin bevorzugt im Bereich von 1:1000 bis 1:1.000.000.

Das Verhältnis von Ruthenium-Verbindung zu anionischem, nichtkoordinierendem Salz liegt im Bereich von 1:1 bis 1:10, bevorzugt liegt das Verhältnis im Bereich von 1:1 bis 1:5, besonders bevorzugt ist ein Verhältnis von 1:5.

Das Verhältnis von Ruthenium-Verbindung zu Lewissäure liegt im Bereich von 1:1 bis 1:10, bevorzugt liegt das Verhältnis in einem Bereich von 1:1 bis 1:5, besonders bevorzugt ist ein Verhältnis von 1:5.

Das Verhältnis von Ruthenium-Verbindung zu Halogenverbindung liegt im Bereich von 1:1 bis 1:333, bevorzugt liegt das Verhältnis in einem Bereich von 1:1 bis 1:33.

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung ausgewählt wird aus Verbindungen, die der Gruppe angehören, die aus den folgenden Elementen besteht (a) Verbindungen abgeleitet aus Formel (I) für die gilt x = 1, y = 1, z = 1 (Formel II), (b) Verbindungen abgeleitet aus Formel (I) für die gilt x = 0, y = 2, z = 1 (Formel III), und (c) Verbindungen abgeleitet aus Formel (I) für die gilt x = 0, y = 1, z = 2 (Formel IV)

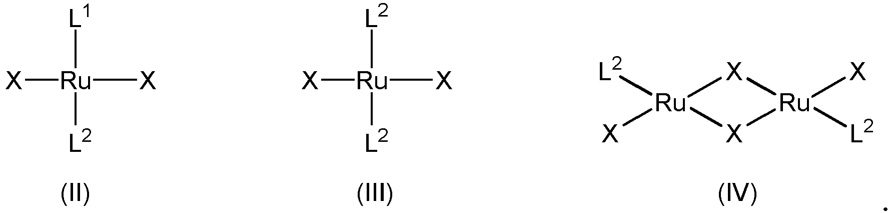

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung eine Verbindung der allgemeinen Formel RuX₂L¹L² (II) ist, worin X gleich ist und Chlor bedeutet, und L² ausgewählt wird aus der Gruppe bestehend aus N-heterocyclischen Carbenen und Phosphanen.

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung eine Verbindung der allgemeinen Formel RuX₂L¹L² (II) ist, worin X gleich ist und Chlor bedeutet, L¹ ausgewählt wird aus der Gruppe bestehend aus Benzol, Toluol, Xylol, Cymen, Trimethylbenzol, Tetramethylbenzol, Hexamethylbenzol, Tetrahydronaphthalin und Naphthalin, und L² ausgewählt wird aus der Gruppe bestehend aus P(Cyclohexyl)₃ und den N-heterocyclischen Carbenen der Formeln VI, VII, VIII, IX, X und XI.

Bevorzugte Ruthenium-Verbindungen der allgemeinen Formel (II) können Tabelle 1 entnommen werden.

**Tabelle 1: Bevorzugte Ruthenium-Verbindungen der allgemeinen Formel (II)**

| Ru-Verbindung | X | L¹ | L² |
|---|---|---|---|
| 1 | Cl | Benzol | P(Phenyl)₃ |
| 2 | Cl | Benzol | P(Cyclohexyl)₃ |
| 3 | Cl | Benzol | VI |
| 4 | Cl | Benzol | VII |
| 5 | Cl | Benzol | VIIII |
| 6 | Cl | Benzol | IX |
| 7 | Cl | Benzol | X |
| 8 | Cl | Benzol | XI |
| 9 | Cl | Cymen | P(Phenyl)₃ |
| 10 | Cl | Cymen | P(Cyclohexyl)₃ |
| 11 | Cl | Cymen | VI |
| 12 | Cl | Cymen | VII |
| 13 | Cl | Cymen | VIIII |
| 14 | Cl | Cymen | IX |
| 15 | Cl | Cymen | X |
| 16 | Cl | Cymen | XI |
| 17 | Cl | Hexamethylbenzol | P(Phenyl)₃ |
| 18 | Cl | Hexamethylbenzol | P(Cyclohexyl)₃ |
| 19 | Cl | Hexamethylbenzol | VI |
| 20 | Cl | Hexamethylbenzol | VII |
| 21 | Cl | Hexamethylbenzol | VIIII |
| 22 | Cl | Hexamethylbenzol | IX |
| 23 | Cl | Hexamethylbenzol | X |
| 24 | Cl | Hexamethylbenzol | XI |

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung eine Verbindung der allgemeinen Formel RuX₂L¹L² (II) ist und ausgewählt wird aus der Gruppe bestehend aus den Verbindungen der Formeln A, B und C

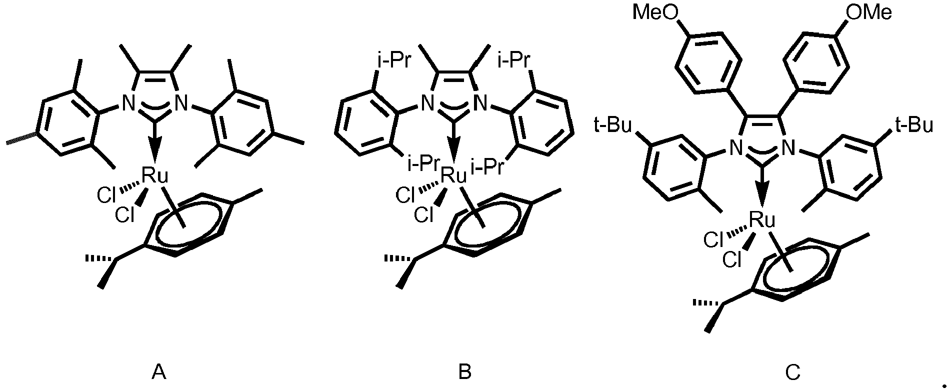

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung eine Verbindung der allgemeinen Formel RuX₂L²₂ (III) ist, worin X gleich ist und Chlor bedeutet, und L² unabhängig voneinander ausgewählt wird aus der Gruppe bestehend aus N-heterocyclischen Carbenen und Phosphanen.

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung eine Verbindung der allgemeinen Formel RuX₂L²₂ (III) ist, worin X gleich ist und Chlor bedeutet, und L² unabhängig voneinander ausgewählt wird aus der Gruppe bestehend aus P(Cyclohexyl)₃, P(Phenyl)₃ und den N-heterocyclischen Carbenen der Formeln VI, VII, VIII, IX, X und XI mit der Maßgabe, dass L² nicht beide P(Phenyl)₃ sind.

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung eine Verbindung der allgemeinen Formel RuX₂L²₂ (III) ist, worin X gleich ist und Chlor bedeutet, und ein L² ausgewählt wird aus der Gruppe bestehend aus P(Cyclohexyl)₃ und P(Phenyl)₃, und das andere L² ausgewählt wird aus der Gruppe bestehend aus den N-heterocyclischen Carbenen der Formeln VI, VII, VIII, IX, X und XI.

Bevorzugte Ruthenium-Verbindungen der allgemeinen Formel (III) können Tabelle 2 entnommen werden.

**Tabelle 2: Bevorzugte Ruthenium-Verbindungen der allgemeinen Formel (III)**

| Ru-Verbindung | X | L² | L² |
|---|---|---|---|
| 25 | Cl | P(Phenyl)₃ | P(Cyclohexyl)₃ |
| 26 | Cl | P(Phenyl)₃ | VI |
| 27 | Cl | P(Phenyl)₃ | VII |
| 28 | Cl | P(Phenyl)₃ | VIII |
| 29 | Cl | P(Phenyl)₃ | IX |
| 30 | Cl | P(Phenyl)₃ | X |
| 31 | Cl | P(Phenyl)₃ | XI |
| 32 | Cl | P(Cyclohexyl)₃ | P(Cyclohexyl)₃ |
| 33 | Cl | P(Cyclohexyl)₃ | VI |
| 34 | Cl | P(Cyclohexyl)₃ | VII |
| 35 | Cl | P(Cyclohexyl)₃ | VIII |
| 36 | Cl | P(Cyclohexyl)₃ | IX |
| 37 | Cl | P(Cyclohexyl)₃ | X |
| 38 | Cl | P(Cyclohexyl)₃ | XI |
| 39 | Cl | VI | VI |
| 40 | Cl | VI | VII |
| 41 | Cl | VI | VIII |
| 42 | Cl | VI | IX |
| 43 | Cl | VI | X |
| 44 | Cl | VI | XI |
| 45 | Cl | VII | VII |
| 46 | Cl | VII | VIII |
| 47 | Cl | VII | IX |
| 48 | Cl | VII | X |
| 49 | Cl | VII | XI |
| 50 | Cl | VIII | VIII |
| 51 | Cl | VIII | IX |
| 52 | Cl | VIII | X |
| 53 | Cl | VIII | XI |
| 54 | Cl | IX | IX |
| 55 | Cl | IX | X |
| 56 | Cl | IX | XI |
| 57 | Cl | X | X |
| 58 | Cl | X | XI |
| 59 | Cl | XI | XI |

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung eine Verbindung der allgemeinen Formel [RuX₂L²]₂ (IV) ist, worin X gleich ist und Chlor bedeutet, und L² unabhängig voneinander ausgewählt wird aus der Gruppe bestehend aus N-heterocyclischen Carbenen und Phosphanen.

Ein weiterer Gegenstand der Erfindung ist ein Verfahren zur Herstellung von Olefinen mittels Metathese umfassend die folgenden Schritte
a) Bereitstellung einer Olefin-Reaktionsmischung enthaltend mindestens ein Olefin,
b) Zugabe einer Ruthenium-Verbindung der allgemeinen Formel [RuX₂L¹ₓL²_{y}]_{z} (I) ist, wobei X = anionischer Ligand; L¹ = neutraler π-bindender Ligand; L² = neutraler Elektronendonor-Ligand; x = 0, 1; y = 1, 2, 3; z = 1, 2,
c) Zugabe einer Lewissäure oder Zugabe einer Lewissäure und eines anionischen, nicht-koordinierenden Salzes,
d) Umsetzung bei Temperaturen in einem Bereich von 30°C bis 140°C,
wobei keine Zugabe eines Alkins oder Alkinols erfolgt, und wobei die Ruthenium-Verbindung eine Verbindung der allgemeinen Formel [RuX₂L²]₂ (IV) ist, worin X gleich ist und Chlor bedeutet, und L² gleich ist und ausgewählt wird aus der Gruppe bestehend aus P(Cyclohexyl)₃ und den N-heterocyclischen Carbenen der Formeln VI, VII, VIII, IX, X und XI.

Bevorzugte Ruthenium-Verbindungen der allgemeinen Formel (IV) können Tabelle 3 entnommen werden.

**Tabelle 3: Bevorzugte Ruthenium-Verbindungen der allgemeinen Formel (IV)**

| Ru-Verbindung | X | L² | L² |
|---|---|---|---|
| 60 | Cl | P(Cyclohexyl)₃ | P(Cyclohexyl)₃ |
| 61 | Cl | P(Cyclohexyl)₃ | VI |
| 62 | Cl | P(Cyclohexyl)₃ | VII |
| 63 | Cl | P(Cyclohexyl)₃ | VIIII |
| 64 | Cl | P(Cyclohexyl)₃ | IX |
| 65 | Cl | P(Cyclohexyl)₃ | X |
| 66 | Cl | P(Cyclohexyl)₃ | XI |
| 67 | Cl | VI | VI |
| 68 | Cl | VI | VII |
| 69 | Cl | VI | VIIII |
| 70 | Cl | VI | IX |
| 71 | Cl | VI | X |
| 72 | Cl | VI | XI |
| 73 | Cl | VII | VII |
| 74 | Cl | VII | VIIII |
| 75 | Cl | VII | IX |
| 76 | Cl | VII | X |
| 77 | Cl | VII | XI |
| 78 | Cl | VIII | VIIII |
| 79 | Cl | VIII | IX |
| 80 | Cl | VIII | X |
| 81 | Cl | VIII | XI |
| 82 | Cl | IX | IX |
| 83 | Cl | IX | X |
| 84 | Cl | IX | XI |
| 85 | Cl | X | X |
| 86 | Cl | X | XI |
| 87 | Cl | XI | XI |

Ein weiterer Gegenstand der Erfindung ist die Verwendung des Verfahrens in Metathesereaktionen, die ausgewählt sind aus der Gruppe bestehend aus Kreuzmetathese (CM), Ringschlussmetathese (RCM), Ringöffnende Metathese (ROM), Ringöffnende Metathesepolymerisation (ROMP) und Acyclische Dien-Metathese (ADMET). Bevorzugt sind die Ringschlussmetathese (RCM) und die Kreuzmetathese (CM).

Im Vergleich zu den in EP0921129A1 beschriebenen Ruthenium- und Osmium-Komplexen für die Olefinmetathese ist in dem vorliegenden erfindungsgemäßen Verfahren keine Isolierung der ionischen Komplexe sowie keine Metall-Carben-Einheit nach der allgemeinen Formel M=C(=C)ₙ=CR¹R² für die Aktivität der Ruthenium-Verbindung notwendig. Daher kann in dem erfindungsgemäßen Verfahren zur Olefinmetathese auf den Einsatz von Alkinolen, wie z.B. den giftigen Propargylalkohol, verzichtet werden. Für die Aktivität der Ruthenium-Verbindung sind außerdem keine Aktivierungsmittel wie photochemische Aktivierung, disubstituierte Cyclopropene, Diazoalkane, oder Alkine notwendig. Weiterhin ist es möglich, die Ruthenium-Verbindung in einer Menge von unter 1 mol-%, bezogen auf das Olefin, einzusetzen.

### Experimenteller Teil

Die folgenden Ruthenium-Verbindungen wurden für die Metathesereaktionen eingesetzt:

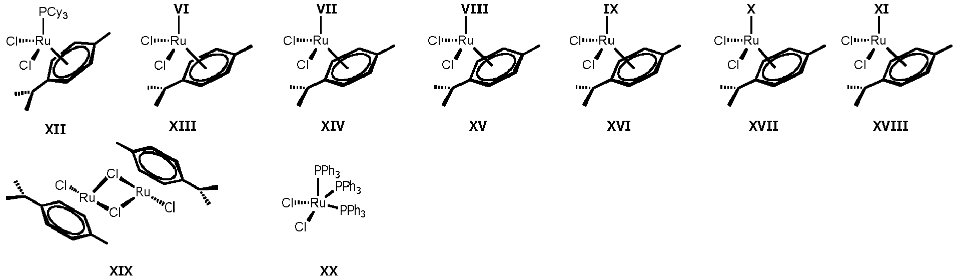

Die nachstehenden Beispiele dienen zur Erläuterung des erfindungsgemäßen Verfahrens, ohne es darauf zu beschränken.

### Allgemeines

Die Reaktionen und die Herstellung von Stammlösungen luftempfindlicher Verbindungen wurden in einer argongefüllten Glove-Box oder in Standard-Schlenkkolben, sowie Schlenkapparaturen durchgeführt. Alle Lösungsmittel wurden getrocknet und über Molsieb gelagert erworben. Die kommerziell erhältlichen Metallsalze und Halogenverbindungen wurden ohne weitere Reinigung verwendet. Die nicht-koordinierenden Salze wurden gegebenenfalls getrocknet und in der Glove-Box gelagert, sowie abgefüllt, sofern diese hygroskopisch sind. Die Olefine und der interne Standard wurden gegebenenfalls entgast und über Molsieb getrocknet. Alle Ringschluss- und Kreuzmetathese-Produkte sind bekannt, sie wurden gegen Hexadecan mittels Gaschromatographie quantitativ bestimmt.

### BEISPIELE 1 BIS 24

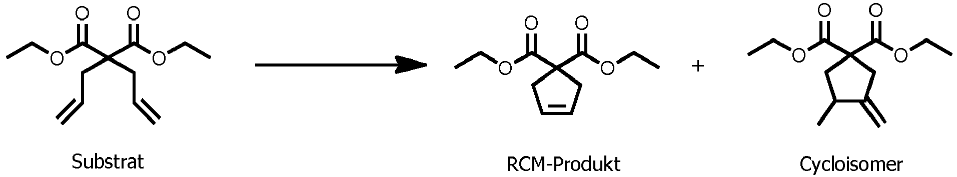

Ruthenium-Verbindungen der allgemeinen Formeln **XII - XX** wurden bezüglich ihrer Aktivität und Selektivität in der Ringschlussmetathese Reaktion von Diethyl diallylmalonate zu Diethyl cyclopent-3-ene-1,1-dicarboxylate gegenüber Cycloisomerisierung Reaktion untersucht. Die Ergebnisse sind in Tabelle 4 zusammengefasst.

### Beispiel 1 (Vergleichsversuch):

Ein 250 mL Dreihalskolben wurde mit 961,2 mg (4,00 mmol) Diethyl diallylmalonat, 452,6 mg (2,00 mmol) Hexadecan und 100 mL trockenem Toluol beladen und die Reaktionsmischung auf 80 °C im Argonstrom erwärmt. In einer Glovebox wurden 25,5 mg (0.04 mmol) Ruthenium-Verbindung **XIV** in 8 mL trockenem Toluol gelöst und zu der warmen Reaktionslösung im 250 mL Kolben überführt. Die Reaktionslösung wurde bei 80 °C für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. Außerdem wurde das Lösungsmittel der Reaktionsmischung abdestilliert und der Rückstand mittels Säulenchromatographie aufgetrennt um 271,0 mg des Diethyl cyclopent-3-ene-1,1-dicarboxylates zu erhalten (isolierte Ausbeute: 32,0 %).

### Beispiele 2 bis 9, 14 und 19 bis 24:

Ein 250 mL Dreihalskolben wurde mit 4,00 mmol Diethyl diallylmalonat, 2,00 mmol Hexadecan, 0 mmol - 0,20 mmol Lewissäure (AlCl₃, PdCl₂(PPh₃)₂, Fe(OAc)₂ oder Fe(acac)₃), 0 mmol - 0,20 mmol NaPF₆ und 100 mL trockenem Lösungsmittel (Toluol oder Xylol) beladen und die Reaktionsmischung auf 80 - 140 °C im Argonstrom erwärmt. In einer Glovebox wurden 0,04 mmol Ruthenium-Verbindung (**XII - XVIII**) in 8 mL trockenem Toluol gelöst und zu der warmen Reaktionslösung im 250 mL Kolben überführt. Die Reaktionslösung wurde bei der gewünschten Reaktionstemperatur für 1-3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. Die konkreten Reaktionsbedingungen und Ergebnisse können Tabelle 4 entnommen werden.

### Beispiele 10 bis 13:

Ein 250 mL Dreihalskolben wurde mit 4,00 mmol Diethyl diallylmalonat, 2,00 mmol Hexadecan, 0,20 mmol Fe(acac)₃, 0,20 mmol NaPF₆, 0,04 mmol 1,2-Dibromcyclohexan und 100 mL trockenem Toluol beladen und die Reaktionsmischung auf 40-100 °C im Argonstrom erwärmt. In einer Glovebox wurden 0,04 mmol Ruthenium Verbindung **XIV** in 8 mL trockenem Toluol gelöst und zu der warmen Reaktionslösung im 250 mL Kolben überführt. Die Reaktionslösung wurde bei der gewünschten Reaktionstemperatur für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. Die konkreten Reaktionsbedingungen und Ergebnisse können Tabelle 4 entnommen werden.

### Beispiele 15 bis 18:

Ein 250 mL Dreihalskolben wurde mit 4,00 mmol Diethyl diallylmalonat, 2,00 mmol Hexadecan, 0,20 mmol Fe(acac)₃, 0,20 mmol NaPF₆, 0,04 mmol 1,2-Dibromcyclohexan und 100 mL trockenem Toluol beladen und die Reaktionsmischung auf 80 °C im Argonstrom erwärmt. In einer Glovebox wurden 0,04 mmol Ruthenium-Verbindung (**XIX** oder **XX**) und 0,04 (1:1) oder 0,08 (1:2) mmol eines entsprechendes Imidazoliumsalzes (**VI***HCl oder **VII***HCl) zusammen mit 0,04 mmol (1:1) oder 0,08 mmol (1:2) NaO-t-Bu in 8 mL trockenem Toluol gelöst und zu der warmen Reaktionslösung im 250 mL Kolben überführt. Die Reaktionslösung wurde bei der gewünschten Reaktionstemperatur für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. Die konkreten Reaktionsbedingungen und Ergebnisse können Tabelle 4 entnommen werden.

**Tabelle 4: Beispiele 1 bis 24.**

| Beispiel | Ru-Verb. | Lewissäure | Salz | Temperatur (°C) | Lösungsmittel | Zeit (h) | Umsatz (%) ^{[b]} | RCM-Produkt (%) ^{[b]} | Cycloisom. (%) ^{[b]} |
|---|---|---|---|---|---|---|---|---|---|
| 1 ^{[a]} | XIV | - | - | 80 | Toluol | 1 | 65 | 33 | 32 |
| 2 ^{[a]} | XIV | - | NaPF₆ | 80 | Toluol | 1 | 76 | 76 | 0 |
| 3 ^{[a],[c]} | XIV | - | NaPF₆ | 80 | Toluol | 1 | 67 | 21 | 43 |
| 4 ^{[a], [d]} | XIV | - | NaPF₆ | 80 | Toluol | 1 | 72 | 63 | 8 |
| 5 ^{[a]} | XIV | AlCl₃ | - | 80 | Toluol | 1 | 34 | 34 | 0 |
| 6 ^{[a]} | XIV | AlCl₃ | NaPF₆ | 80 | Toluol | 1 | 94 | 94 | 0 |
| 7 ^{[a]} | XIV | PdCl₂ (PPh₃)₂ | NaPF₆ | 80 | Toluol | 1 | 64 | 61 | 0 |
| 8 ^{[a]} | XIV | Fe(OAc)₂ | NaPF₆ | 80 | Toluol | 3 | >99 | 97 | 3 |
| 9 ^{[a]} | XIV | Fe(acac)₃ | - | 80 | Toluol | 1 | 48 | 46 | 0 |
| 10 ^{[a],[e]} | XIV | Fe(acac)₃ | NaPF₆ | 40 | Toluol | 3 | 12 | 10 | 1 |
| 11 ^{[a],[e]} | XIV | Fe(acac)₃ | NaPF₆ | 60 | Toluol | 3 | 43 | 40 | 1 |
| 12 ^{[a],[e]} | XIV | Fe(acac)₃ | NaPF₆ | 70 | Toluol | 3 | 78 | 78 | 0 |
| 13 ^{[a],[e]} | XIV | Fe(acac)₃ | NaPF₆ | 100 | Toluol | 3 | 76 | 76 | 0 |
| 14 ^{[a]} | XIV | Fe(acac)₃ | NaPF₆ | 140 | Xylol | 3 | 49 | 15 | 15 |
| 15 ^{[a],[e]} | XIX/VI (1:2) | Fe(acac)₃ | NaPF₆ | 80 | Toluol | 3 | 46 | 43 | 2 |
| 16 ^{[a],[c]} | XIX/VII (1:1) | Fe(acac)₃ | NaPF₆ | 80 | Toluol | 3 | 24 | 23 | 1 |
| 17 ^{[a],[e]} | XIX/VII (1:2) | Fe(acac)₃ | NaPF₆ | 80 | Toluol | 3 | 56 | 52 | 3 |
| 18 ^{[a],[e]} | XX/VII (1:2) | Fe(acac)₃ | NaPF₆ | 80 | Toluol | 3 | 4 | 4 | 0 |
| 19 ^{[a]} | XIII | Fe(acac)₃ | NaPF₆ | 80 | Toluol | 1 | 52 | 48 | 3 |
| 20 ^{[a]} | XVI | Fe(acac)₃ | NaPF₆ | 80 | Toluol | 1 | 24 | 22 | 0 |
| 21 ^{[a]} | XV | Fe(acac)₃ | NaPF₆ | 80 | Toluol | 1 | 22 | 14 | 3 |
| 22 ^{[a]} | XVII | Fe(acac)₃ | NaPF₆ | 80 | Toluol | 1 | 47 | 44 | 0 |
| 23 ^{[a]} | XVIII | Fe(acac)₃ | NaPF₆ | 80 | Toluol | 1 | 4 | 3 | 0 |
| 24 ^{[a]} | XII | Fe(acac)₃ | NaPF₆ | 80 | Toluol | 1 | 4 | 3 | 0 |

| | | | | | | | | | |
|---|---|---|---|---|---|---|---|---|---|
| [a] Reaktionsbedingungen: Diethyl diallylmalonat (4 mmol), Ru-Verbindung (1 mol%), NaPF₆ (5 mol%), und Lewissäure (5 mol%), Lösungsmittel (100 mL), Argonstrom; [b] Bestimmt durch GC mit internem Standard Hexadecan; [c] NaPF₆ (1 mol%); [d] NaPF₆ (10 mol%); [e] 1,2-Dibromcyclohexan (1 mol%) zugesetzt. | | | | | | | | | |

### BEISPIELE 25 BIS 41

### Einfluss von Halogenverbindungen

Das Modellkatalysatorsystem bestehend aus **XIV,** NaPF₆ und Fe(acac)₃ wurde bezüglich seiner Aktivität und Selektivität in Abhängigkeit der Anwesenheit von ausgewählten Halogenverbindungen (**H1** - **H17**) untersucht.

### Beispiele 25 bis 41:

Ein 50 mL Dreihalskolben wurde mit 1,00 mmol Diethyl diallylmalonate, 0,50 mmol Hexadecan, 0,05 mmol Fe(acac)₃, 0,05 mmol NaPF₆, 0,01 mmol Halogenverbindung (**H1** bis **H17**) und 21 mL trockenem Toluol beladen und die Reaktionsmischung auf 80 °C im Argonstrom erwärmt. In einer Glovebox wurden 0,01 mmol **XIV** in 4 mL trockenem Toluol gelöst und zu der warmen Reaktionslösung im 50 mL Kolben überführt. Die Reaktionslösung wurde bei 80°C für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. Die konkreten Reaktionsbedingungen und Ergebnisse können Tabelle 5 entnommen werden.

**Tabelle 5: Einfluss von Halogenverbindungen.**

| Beispiel ^{[a]} | Halogenverbindung | Umsatz (%) ^{[b]} | RCM-Produkt (%) ^{[b]} | Ausbeute Cycloisomer (%) ^{[b]} |
|---|---|---|---|---|
| 25 | Benzylchlorid **(H1)** | 42 | 35 | 4 |
| 26 | Benzylbromid (**H2**) | 41 | 38 | 1 |
| 27 | (1,2-Dibromethyl)benzol (**H3**) | 50 | 42 | 4 |
| 28 | Cyclohexyliodid (**H4**) | 34 | 34 | 0 |
| 29 | Dibromomethan (**H5**) | 70 | 58 | 3 |
| 30 | 1,2-Dibromocyclohexane (**H6**) | 99 | 87 | 3 |
| 31 | Cyclohexylbromid (**H7**) | 71 | 68 | 3 |
| 32 | 1,2-Dibromethan (**H8**) | 76 | 71 | 4 |
| 33 | 1,2-Dibrom-4,5-dimethylbenzol **(H9)** | 80 | 74 | 2 |
| 34 | 1,2-Diiodbenzol (**H10**) | 86 | 84 | 2 |
| 35 | 1-Brom-2-iodbenzol **(H11)** | 74 | 70 | 4 |
| 36 | 2-Bromstyrol (**H12**) | 84 | 79 | 1 |
| 37 | (2-Bromoethyl)benzol (**H13**) | 74 | 71 | 2 |
| 38 | (3-Bromopropyl)benzol (**H14**) | 72 | 70 | 2 |
| 39 | Tetrabutylammoniumbromid (**H15**) | 82 | 80 | 2 |
| 40 | KBr (**H16**) | 60 | 50 | 2 |
| 41 | KI (**H17**) | 63 | 53 | 2 |

| | | | | |
|---|---|---|---|---|
| [a] Reaktionsbedingungen: Diethyl diallylmalonat (1 mmol), (p-cymene) Ru (Me₂IMes)Cl₂ (**XIV**) (1 mol%), NaPF₆ (5 mol%), Fe(acac)₃ (5 mol%), Halogenverbindung (1 mol%), Toluol (25 mL), Argonstrom, 80°C, 3h; [b] bestimmt durch GC mit internem Standard Hexadecan. | | | | |

### BEISPIEL 42

### Synthese von Diethyl 3-methylcyclohex-3-ene-1,1-dicarboxylate

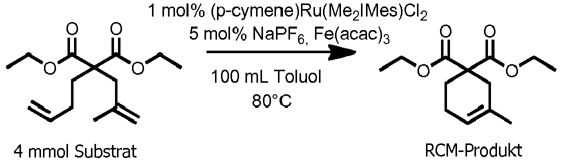

Ein 250 mL Dreihalskolben wurde mit 4,00 mmol Diethyl 2-(but-3-enyl)-2-(2-methylallyl)malonat, 2,00 mmol Hexadecan, 0,2 mmol Fe(acac)₃, 0,2 mmol NaPF₆ und 100 mL trockenem Toluol beladen und die Reaktionsmischung auf 80 °C im Argonstrom erwärmt. In einer Glovebox wurden 0,04 mmol **XIV** in 8 mL trockenem Toluol gelöst und zu der warmen Reaktionslösung im 250 mL Kolben überführt. Die Reaktionslösung wurde bei der 80°C für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. In Gegenwart von **XIV,** NaPF₆ und Fe(acac)₃ wurden ein Umsatz von 50 % und eine RCM-Ausbeute von 24 % erreicht.

### BEISPIEL 43

### Synthese von 1-Tosyl-2,5-dihydro-1H-pyrrol

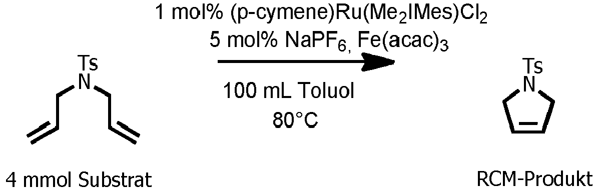

Ein 250 mL Dreihalskolben wurde mit 4,00 mmol *N*,*N*-diallyl-4-methylbenzolsufonamid, 2,00 mmol Hexadecan, 0,2 mmol Fe(acac)₃, 0,2 mmol NaPF₆ und 100 mL trockenem Toluol beladen und die Reaktionsmischung auf 80 °C im Argonstrom erwärmt. In einer Glovebox wurden 0,04 mmol **XIV** in 8 mL trockenem Toluol gelöst und zu der warmen Reaktionslösung im 250 mL Kolben überführt. Die Reaktionslösung wurde bei 80°C für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. In Gegenwart von **XIV,** NaPF₆ und Fe(acac)₃ wurden ein Umsatz von 14 % und eine RCM-Ausbeute von 12 % erreicht.

### BEISPIEL 44

### Synthese von 1-Benzyloxycyclohex-3-en

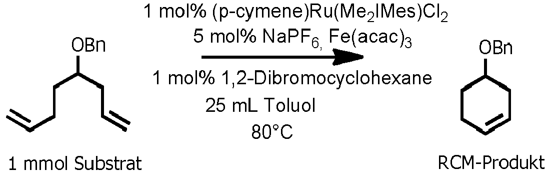

Ein 50 mL Dreihalskolben wurde mit 1,00 mmol 4-Benzyloxy-octa-1,7-dien, 0,50 mmol Hexadecan, 0,05 mmol Fe(acac)₃, 0,05 mmol NaPF₆, 0,01 mmol 1,2-Dibromcyclohexan und 21 mL trockenem Toluol beladen und die Reaktionsmischung auf 80 °C im Argonstrom erwärmt. In einer Glovebox wurden 0,01 mmol **XIV** in 4 mL trockenem Toluol gelöst und zu der warmen Reaktionslösung im 250 mL Kolben überführt. Die Reaktionslösung wurde bei 80°C für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. In Gegenwart von **XIV,** NaPF₆, Fe(acac)₃ und 1,2-Dibromcyclohexan wurden ein Umsatz von 85 % und eine RCM-Ausbeute von 41 % erreicht.

### BEISPIEL 45

### Synthese von 1-Benzyloxycyclopent-3-en

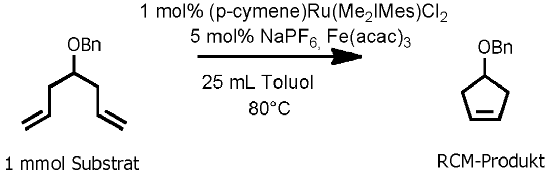

Ein 50 mL Dreihalskolben wurde mit 1,00 mmol 4-Benzyloxyhepta-1,7-dien, 0,50 mmol Hexadecan, 0,05 mmol Fe(acac)₃, 0,05 mmol NaPF₆, 0,01 mmol 1,2-Dibromcyclohexan und 21 mL trockenem Toluol beladen und die Reaktionsmischung auf 80 °C im Argonstrom erwärmt. In einer Glovebox wurden 0,01 mmol **XIV** in 4 mL trockenem Toluol gelöst und zu der warmen Reaktionslösung im 250 mL Kolben überführt. Die Reaktionslösung wurde bei 80°C für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. In Gegenwart von **XIV,** NaPF₆, Fe(acac)₃ und 1,2-Dibromcyclohexan wurden ein Umsatz von 90 % und eine RCM-Ausbeute von 81 % erreicht.

### BEISPIEL 46

### Synthese von 1-Benzyloxy-1-methyl-cyclopent-3-en

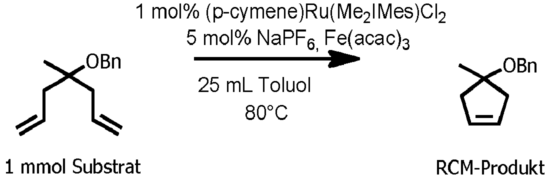

Ein 50 mL Dreihalskolben wurde mit 1,00 mmol 4-Benzyloxy-4-methylhepta-1,7-dien, 0,50 mmol Hexadecan, 0,05 mmol Fe(acac)₃, 0,05 mmol NaPF₆, und 21 mL trockenem Toluol beladen und die Reaktionsmischung auf 80 °C im Argonstrom erwärmt. In einer Glovebox wurden 0,01 mmol **XIV** in 4 mL trockenem Toluol gelöst und zu der warmen Reaktionslösung im 50 mL Kolben überführt. Die Reaktionslösung wurde bei der gewünschten Reaktionstemperatur für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. In Gegenwart von **XIV,** NaPF₆ und Fe(acac)₃ wurden ein Umsatz von 95 % und eine RCM-Ausbeute von 94 % erreicht.

### BEISPIELE 47 UND 48

### Synthese von 2,5-Dihydrobenzo[b]oxepin

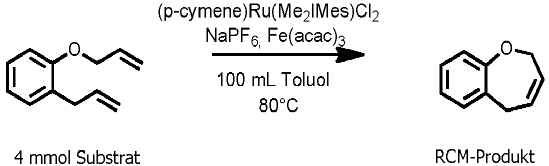

Beispiel 47: Ein 250 mL Dreihalskolben wurde mit 4,00 mmol 1-Allyl-2-(allyloxy)benzol, 2,00 mmol Hexadecan, 0,2 mmol Fe(acac)₃, 0,2 mmol NaPF₆ und 100 mL trockenem Toluol beladen und die Reaktionsmischung auf 80 °C im Argonstrom erwärmt. In einer Glovebox wurden 0,04 mmol **XIV** in 8 mL trockenem Toluol gelöst und zu der warmen Reaktionslösung im 250 mL Kolben überführt. Die Reaktionslösung wurde bei der gewünschten Reaktionstemperatur für 3 h im Argonstrom gerührt. Nach Beendigung der Reaktion wurden 0,1 mL der Reaktionslösung im Argongegenstrom in ein GC-Vial gegeben, in 1,5 mL Essigsäureethylester gelöst und mittels eines HP 6890 Gaschromatographen (GC) analysiert. In Gegenwart von 1 mol% **XIV,** NaPF₆ und Fe(acac)₃ wurden ein Umsatz von 94 % und eine RCM-Ausbeute von 85 % erreicht.

### Beispiel 48:

Ein 250 mL Dreihalskolben wurde mit 4,00 mmol 1-Allyl-2-(allyloxy)benzol, 2,00 mmol Hexadecan, 0,02 mmol Fe(acac)₃, 0,02 mmol NaPF₆ und 100 mL trockenem Toluol beladen und die Reaktionsmischung auf 80 °C im Argonstrom erwärmt. In einer Glovebox wurden 0,004 mmol **XIV** in 8 mL trockenem Toluol gelöst und zu der warmen Reaktionslösung im 250 mL Kolben überführt. Die Reaktionslösung wurde bei 80°C für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. In Gegenwart von 0,01 mol% **XIV,** NaPF₆ und Fe(acac)₃ wurden ein Umsatz von 94 % und eine RCM-Ausbeute von 81 % erreicht.

### BEISPIEL 49

### Synthese von (E)-1,2-Diphenylethen (Kreuzmetathese)

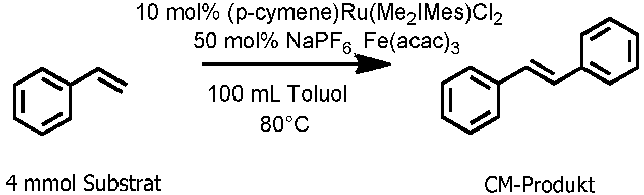

Ein 250 mL Dreihalskolben wurde mit 4,00 mmol Styrol, 2,00 mmol Hexadecan, 2,0 mmol Fe(acac)₃, 2,0 mmol NaPF₆ und 80 mL trockenem Toluol beladen und die Reaktionsmischung auf 80 °C im Argonstrom erwärmt. In einer Glovebox wurden 0,4 mmol **XIV** in 20 mL trockenem Toluol gelöst und zu der warmen Reaktionslösung im 250 mL Kolben überführt. Die Reaktionslösung wurde bei 80°C für 3 h im Argonstrom gerührt. Nach Beendigung der Reaktion wurden 0,1 mL der Reaktionslösung in 1,5 mL Essigsäureethylester gelöst und mittels eines HP 6890 Gaschromatographen (GC) analysiert. In Gegenwart von **XIV,** NaPF₆ und Fe(acac)₃ wurden ein Umsatz von 23 % und eine CM-Ausbeute von 13 % erreicht.

## Patentansprüche

1. Verfahren zur Herstellung von Olefinen mittels Metathese umfassend die folgenden Schritte
a. Bereitstellung einer Olefin-Reaktionsmischung enthaltend mindestens ein Olefin,
b. Zugabe einer Ruthenium-Verbindung der allgemeinen Formel [RuX₂L¹ₓL²_{y}]_{z} (I), wobei X = anionischer Ligand; L¹ = neutraler π-bindender Ligand; L² = neutraler Elektronendonor-Ligand; x = 0, 1; y = 1, 2, 3; z = 1, 2,
c. Zugabe einer Lewissäure oder Zugabe einer Lewissäure und eines anionischen, nicht-koordinierenden Salzes,
d. Umsetzung bei Temperaturen in einem Bereich von 30°C bis 140°C,
wobei keine Zugabe eines Alkins oder Alkinols erfolgt.

2. Verfahren nach Anspruch 1, wobei X gleich ist und Chlor ist, und L² ausgewählt wird aus der Gruppe bestehend aus Stickstoffbasen, Phosphanen, Phosphiniten, Phosphoniten, Phosphiten und Arsanen.

3. Verfahren nach Anspruch 2, wobei L¹ ausgewählt wird aus der Gruppe bestehend aus Benzol, Toluol, Xylol, Cymen, Trimethylbenzol, Tetramethylbenzol, Hexamethylbenzol, Tetrahydronaphthalin und Naphthalin, und L² ausgewählt wird aus der Gruppe bestehend aus N-heterocyclischen Carbenen und Phosphanen.

4. Verfahren nach Anspruch 3, wobei L² ausgewählt wird aus der Gruppe bestehend aus P(Phenyl)₃, P(Cyclohexyl)₃, und N-heterocyclischen Carbenen der Formeln VI, VII, VIII, IX, X und XI.

5. Verfahren nach einem der Ansprüche 1 bis 4, wobei die Ruthenium-Verbindung ausgewählt wird aus Verbindungen, die der Gruppe angehören, die aus den folgenden Elementen besteht (a) Verbindungen abgeleitet aus Formel (I) für die gilt x = 1, y = 1, z = 1 (Formel II), (b) Verbindungen abgeleitet aus Formel (I) für die gilt x = 0, y = 2, z = 1 (Formel III), und (c) Verbindungen abgeleitet aus Formel (I) für die gilt x = 0, y = 1, z = 2 (Formel IV)

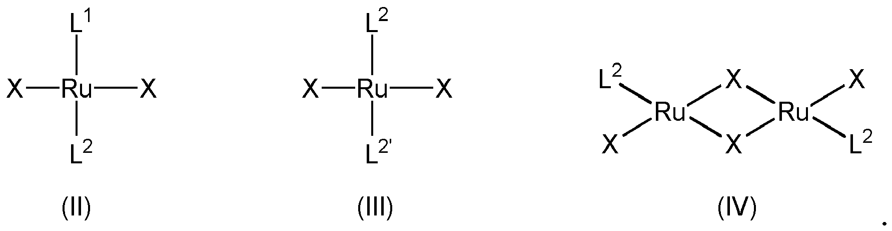

6. Verfahren nach Anspruch 5, wobei die Ruthenium-Verbindung eine Verbindung der allgemeinen Formel RuX₂L¹L² (II) ist, worin X gleich ist und Chlor bedeutet, und L² ausgewählt wird aus der Gruppe bestehend aus N-heterocyclischen Carbenen und Phosphanen.

7. Verfahren nach Anspruch 6, wobei L¹ ausgewählt wird aus der Gruppe bestehend aus Benzol, Toluol, Xylol, Cymen, Trimethylbenzol, Tetramethylbenzol, Hexamethylbenzol, Tetrahydronaphthalin und Naphthalin, und L² ausgewählt wird aus der Gruppe bestehend aus P(Cyclohexyl)₃ und den N-heterocyclischen Carbenen der Formeln VI, VII, VIII, IX, X und XI.

8. Verfahren nach Anspruch 7, wobei die Ruthenium-Verbindung ausgewählt wird aus der Gruppe bestehend aus den Verbindungen der Formeln A, B und C.

9. Verfahren nach Anspruch 5, wobei die Ruthenium-Verbindung eine Verbindung der allgemeinen Formel RuX₂L²₂ (III) ist, worin X gleich ist und Chlor bedeutet, und L² unabhängig voneinander ausgewählt wird aus der Gruppe bestehend aus N-heterocyclischen Carbenen und Phosphanen.

10. Verfahren nach Anspruch 9, wobei L² unabhängig voneinander ausgewählt wird aus der Gruppe bestehend aus P(Cyclohexyl)₃, P(Phenyl)₃ und den N-heterocyclischen Carbenen der Formeln VI, VII, VIII, IX, X und XI.

11. Verfahren nach Anspruch 10, wobei ein L² ausgewählt wird aus der Gruppe bestehend aus P(Cyclohexyl)₃ und P(Phenyl)₃, und das andere L² ausgewählt wird aus der Gruppe bestehend aus den N-heterocyclischen Carbenen der Formeln VI, VII, VIII, IX, X und XI.

12. Verfahren nach Anspruch 5, wobei die Ruthenium-Verbindung eine Verbindung der allgemeinen Formel [RuX₂L²]₂ (IV) ist, worin X gleich ist und Chlor bedeutet, und L² unabhängig voneinander ausgewählt wird aus der Gruppe bestehend aus N-heterocyclischen Carbenen und Phosphanen.

13. Verfahren nach Anspruch 12, wobei L² gleich ist und ausgewählt wird aus der Gruppe bestehend aus P(Cyclohexyl)₃ und den N-heterocyclischen Carbenen der Formeln VI, VII, VIII, IX, X und XI.

14. Verfahren nach einem der Ansprüche 1 bis 13, wobei das anionische, nicht-koordinierende Salz ausgewählt wird aus der Gruppe bestehend aus einem Natrium-, Kalium-, Caesium-, Barium-, Calcium- und Magnesium-Salz von PF₆⁻, BF₄⁻, BH₄⁻, F₃CSO₃⁻, H₃CSO₃⁻, ClO₄⁻, SO₄²⁻, HSO₄⁻, NO₃⁻, PO₄³⁻, HPO₄²⁻, H₂PO₄⁻, CF₃COO⁻, B(C₆F₅)₄⁻, B[3,5-(CF₃)₂C₆H₃]₄⁻, RSO₃⁻, und R'COO⁻, wobei R, R' unabhängig voneinander ausgewählt sind aus der Gruppe bestehend aus (C₁-C₂₀)-Alkyl und (C₆-C₁₄)-Aryl.

15. Verfahren nach einem der Ansprüche 1 bis 14, wobei die Lewissäure ausgewählt wird aus der Gruppe bestehend aus Aluminium-, Bor-, Chrom-, Kobalt-, Eisen-, Kupfer-, Magnesium-, Lanthan-, Mangan-, Nickel-, Palladium- oder Zink-Salzen von Cl⁻, Br⁻, I⁻, PF₆⁻, BF₄⁻, CF₃COO⁻, B(C₆F₅)₄⁻, B[3,5-(CF₃)₂C₆H₃4⁻, R^{x}COO⁻, (R^{y}COCHCOR^{z})⁻, wobei R^{x}, R^{y}, R^{z} unabhängig voneinander ausgewählt sind aus der Gruppe bestehend aus (C₁-C₂₀)-Alkyl und (C₆-C₁₄)-Aryl.

16. Verfahren nach einem der Ansprüche 1 bis 15, wobei in Schritt c) zusätzlich eine Halogenverbindung zugegeben wird.

17. Verfahren nach Anspruch 16, wobei die Halogenverbindung ausgewählt wird aus der Gruppe bestehend aus Kaliumiodid, Kaliumbromid, Tetrabutylammoniumbromid, 1,2-Dibromocyclohexane, 1,2-Bromocyclohexane, 1,2-Dibromoethane, 1,2-Dibromo-4,5-dimethylbenzol, 1,2-Diiodobenzol, 1-Bromo-2-iodo-benzol, 2-Bromo-styrol, (2-Bromoethyl)benzol, und (3-Bromopropyl)benzol.

18. Verfahren nach einem der Ansprüche 1 bis 17, wobei das Verhältnis von Ruthenium-Verbindung zu Olefin im Bereich von 1:10 bis 1:1.000.000 liegt.

19. Verfahren nach einem der Ansprüche 1 bis 18, wobei das Verhältnis von Ruthenium-Verbindung zu anionischem, nichtkoordinierendem Salz im Bereich von 1:1 bis 1:10 liegt.

20. Verfahren nach einem der Ansprüche 1 bis 19, wobei das Verhältnis von Ruthenium-Verbindung zu Lewissäure im Bereich von 1:1 bis 1:10 liegt.

21. Verfahren nach einem der Ansprüche 16 bis 20, wobei das Verhältnis von Ruthenium-Verbindung zu Halogenverbindung im Bereich von 1:1 bis 1:333 liegt.

22. Verwendung des Verfahrens nach einem der Ansprüche 1-21 in Metathesereaktionen, die ausgewählt sind aus der Gruppe bestehend aus Kreuzmetathese (CM), Ringschlussmetathese (RCM), Ringöffnende Metathese (ROM), Ringöffnende Metathesepolymerisation (ROMP) und Acyclische Dien-Metathese (ADMET).
